(19) Europäisches Patentamt
European Patent Office
Office européen des brevets

(11) **EP 1 613 767 B1**

(12) **EUROPEAN PATENT SPECIFICATION**

(45) Date of publication and mention of the grant of the patent:
**03.10.2012 Bulletin 2012/40**

(51) Int Cl.:
*C12Q 1/68* (2006.01)  *G01N 33/574* (2006.01)

(21) Application number: **03816604.7**

(22) Date of filing: **08.04.2003**

(86) International application number:
**PCT/JP2003/004458**

(87) International publication number:
**WO 2004/090163 (21.10.2004 Gazette 2004/43)**

(54) **METHOD OF DEFINING THE DIFFERENTIATION GRADE OF TUMOR**

METHODE ZUR BESTIMMUNG DES DIFFERZIERUNGSGRADES VON TUMOREN

PROCEDE DE DEFINITION DU DEGRE DE DIFFERENCIATION D'UNE TUMEUR

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(43) Date of publication of application:
**11.01.2006 Bulletin 2006/02**

(73) Proprietor: **F.Hoffmann-La Roche AG**
**4070 Basel (CH)**

(72) Inventors:
• **OKA, Masaaki**
  **Ube-shi, Yamaguchi 755-0093 (JP)**
• **HAMAMOTO, Yoshihiko**
  **Ube-shi, Yamaguchi 755-0001 (JP)**
• **IIZUKA, Norio**
  **Ube-shi, Yamaguchi 755-0151 (JP)**
• **OKABE, Hisafumi**
  **Yokohama-shi, Kanagawa 247-0022 (JP)**
• **HAMADA, Kenji**
  **Grandcity Shounan-Enoshima 506**
  **Kanagawa 251-0032 (JP)**

(74) Representative: **Vossius & Partner**
**Siebertstrasse 4**
**81675 München (DE)**

(56) References cited:
**WO-A-03/010337**

• **MIDORIKAWA YUTAKA ET AL: "Identification of genes associated with dedifferentiation of hepatocellular carcinoma with expression profiling analysis" JAPANESE JOURNAL OF CANCER RESEARCH, vol. 93, no. 6, June 2002 (2002-06), pages 636-643, XP009024186 ISSN: 0910-5050**
• **DELPUECH OONA ET AL: "Identification, using cDNA macroarray analysis, of distinct gene expression profiles associated with pathological and virological features of hepatocellular carcinoma" ONCOGENE, vol. 21, no. 18, 25 April 2002 (2002-04-25), pages 2926-2937, XP002267966 ISSN: 0950-9232**
• **SHIROTA YUKIHIRO ET AL: "Identification of differentially expressed genes in hepatocellular carcinoma with cDNA microarrays" HEPATOLOGY, vol. 33, no. 4, April 2001 (2001-04), pages 832-840, XP009024178 ISSN: 0270-9139**
• **IIZUKA N. ET AL.: "Comparison of Gene Expression Profiles between Hepatitis B Virus- and Hepatitis C Virus-infected Hepatocellular Carcinoma by Oligonucleotide Microarray Data on the Basis of a Supervised Learning Method", CANCER RESEARCH, vol. 62, 2002, pages 3939-3944,**
• **TAMAYO P. ET AL.: "Interpreting patterns of gene expression with a self-organizing maps: Methods and application to hematopoietic differentiation", PROC. NATL. ACAD. SCI. USA, vol. 96, 1999, pages 2907-2912,**
• **TÖRÖNEN P. ET AL.: "Analysis of gene expression data using self-organizing maps", FEBS LETTERS, vol. 451, 1999, pages 142-146,**

**Description**

Technical Field

The present invention relates to a method of defining the differentiation grade of tumor. More particularly, the present invention relates to a method of defining the differentiation grade of tumor by selecting genes and/or proteins whose expression level correlates with each differentiation grade of hepatocellular carcinoma (HCC), measuring the expression of the genes and/or proteins of human tumor tissues in each differentiation grade.

There is also disclosed the use of these genes and/or proteins for diagnosing the differentiation grade of HCC and for screening anti-cancer agents for HCC treatment.

There is also disclosed a kit for performing the method of the present invention comprising DNA chips, oligonucleotide chips, protein chips, peptides, antibodies, probes and primers that are necessary for DNA microarrays, oligonucleotide microarrays, protein arrays, northern blotting, in situ hybridization, RNase protection assays, western blotting, ELISA assays, reverse transcription polymerase-chain reaction (hereinafter referred to as RT-PCR) to examine the expression of the genes and/or proteins whose expression level correlates with the differentiation grade of tumor.

Background Art

Cancer is the major causative of death in the world. Particularly, hepatocellular carcinoma (HCC) is one of the most common cancers worldwide, which represents a major international health problem because of its increasing incidence in many countries (Schafer, D.F. and Sorrell, M.F. Hepatocellular carcinoma, Lancet 353, 1253-1257 (1999), Colombo, M. Hepatitis C virus and hepatocellular carcinoma, Semin. Liver Dis. 19, 263-269 (1999), and Okuda, K. Hepatocellular carcinoma, J. Hepatol. 32, 225-237 (2000)). Chronic hepatitis C virus (HCV) infection is one of the major risk factors for HCC as well as hepatitis B virus (HBV) infection, alcohol consumption, and several carcinogens such as aflatoxin B1 (Okuda, K. Hepatocellular carcinoma, J. Hepatol. 32, 225-237 (2000)). Several therapies have been adopted for the treatment of HCC. Those include surgical resection, radiotherapy, chemotherapy, and biological therapy including hormonal and gene therapy. However, none of these therapies could cure the disease. One of the major problems of HCC treatment is that characteristics of cancer cells change during the development and progression of the disease. Particularly, changes in the differentiation grade of tumor cells are apparent and frequent. Such changes alter the ability of tumor cells to invade and metastasize and also the sensitivity of cancer cells to different therapies, causing resistance to anti-cancer agents. If the changes in the characteristics of cancer cells are precisely diagnosed and managed, cancer therapy will be more effective.

Previous studies suggested the involvement of tumor suppressor genes and oncogenes such as *p53, β-catenin,* and *AXIN1* genes in hepatocarcinogenesis (Okabe, H. , Satoh, S. , Kato, T., Kitahara, O., Yanagawa, R., Yamaoka, Y., Tsunoda, T., Furukawa, Y., and Nakamura, Y. Genome-wide analysis of gene expression in human hepatocellular carcinomas using cDNA microarray: identification of genes involved in viral carcinogenesis and tumor progression, Cancer Res. 61, 2129-2137 (2001)). It has also been suggested that the development of HCV-associated HCC can be characterized by the pathological evolution from early to advanced tumor, which correlates with dedifferentiation of cancer cells (Kojiro, M. Pathological evolution of early hepatocellular carcinoma, Oncology 62, 43-47 (2002)). Particularly after introduction of DNA microarray technologies into medical science (Schena, M., Shalon, D., Davis, R.W., and Brown, P.O. Quantitative monitoring of gene expression patterns with a complementary DNA microarray, Science 270, 467-470 (1995), DeRisi, J., Penland, L., Brown, P.O., Bittner, M.L., Meltzer, P.S., Ray, M., Chen, Y. , Su, Y . A . , and Trent, J.M. Use of a cDNA microarray to analyse gene expression patterns in human cancer, Nat. Genet. 14, 457-460 (1996)), many studies showed gene-expression patterns relating to some aspects of HCC (Lau, W.Y., Lai, P.B., Leung, M.F., Leung, B.C., Wong, N., Chen, G., Leung, T.W., and Liew, C.T. Differential gene expression of hepatocellular carcinoma using cDNA microarray analysis, Oncol. Res. 12, 59-69 (2000), Tackels-Horne, D., Goodman, M.D., Williams, A.J., Wilson, D.J., Eskandari, T., Vogt, L.M., Boland, J.F., Scherf, U., and Vockley, J.G. Identification of differentially expressed genes in hepatocellular carcinoma and metastatic liver tumors by oligonucleotide expression profiling, Cancer 92, 395-405 (2001), Xu, L., Hui, L., Wang, S., Gong, J., Jin, Y., Wang, Y., Ji, Y., Wu, X., Han, Z., and Hu, G. Expression profiling suggested a regulatory role of liver-enriched transcription factors in human hepatocellular carcinoma, Cancer Res. 61, 3176-3681 (2001), Xu, X.R., Huang, J., Xu, Z.G., Qian, B.Z., Zhu, Z. D. , Yan, Q., Cai, T., Zhang, X. , Xiao, H.S., Qu, J. , Liu, F. , Huang, Q.H., Cheng, Z. H . , Li, N. G. , Du, J.J., Hu, W., Shen, K.T., Lu, G., Fu, G., Zhong, M. , Xu, S. H . , Gu, W.Y., Huang, W. , Zhao, X. T. , Hu, G. X. , Gu, J. R. , Chen, Z., and Han, Z.G. Insight into hepatocellular carcinogenesis at transcriptome level by comparing gene expression profiles of hepatocellular carcinoma with those of corresponding non-cancerous liver, Proc. Natl. Acad. Sci. U.S.A. 98, 15089-15094 (2001), Okabe, H., Satoh, S., Kato, T., Kitahara, O., Yanagawa, R., Yamaoka, Y., Tsunoda, T., Furukawa, Y., and Nakamura, Y. Genome-wide analysis of gene expression in human hepatocellular carcinomas using cDNA microarray: identification of genes involved in viral carcinogenesis and tumor progression, Cancer Res. 61, 2129-2137 (2001), Shirota, Y., Kaneko, S., Honda, M., Kawai, H.F., and Kobayashi,

K. Identification of differentiallyexpressed genes in hepatocellular carcinoma with cDNA microarrays, Hepatology 33, 832-840 (2001), Delpuech, O., Trabut, J.B., Carnot, F., Feuillard, J., Brechot, C., and Kremsdorf, D. Identification, using cDNA macroarray analysis, of distinct gene expression profiles associated with pathological and virological features of hepatocellular carcinoma, Oncogene 21, 2926-2937 (2002), Iizuka, N. , Oka, M. , Yamada-Okabe, H., Mori, N., Tamesa, T., Okada, T., Takemoto, T., Tangoku, A., Hamada, K., Nakayama, H., Miyamoto, T., Uchimura, S., and Hamamoto, Y. Comparison of gene expression profiles between hepatitis B virus- and hepatitis C virus-infected hepatocellular carcinoma by oligonucleotide microarray data based on a supervised learning method, Cancer Res. 62, 3939-3944 (2002), and Midorikawa, Y., Tsutsumi, S., Taniguchi, H. , Ishii, M., Kobune, Y. , Kodama, T. , Makuuchi, M. , and Aburatani, H. Identification of genes associated with dedifferentiation of hepatocellular carcinoma with expression profiling analysis, Jpn. J. Cancer Res. 93, 636-643 (2002)). Among them, two studies profiled gene expression of HCC in relation to its development (Okabe, H., Satoh, S., Kato, T., Kitahara, O., Yanagawa, R. , Yamaoka, Y., Tsunoda, T. , Furukawa, Y., and Nakamura, Y. Genome-wide analysis of gene expression in human hepatocellular carcinomas using cDNA microarray: identification of genes involved in viral carcinogenesis and tumor progression, Cancer Res. 61, 2129-2137 (2001) and Midorikawa, Y., Tsutsumi, S. , Taniguchi, H., Ishii, M. , Kobune, Y. , Kodama, T. , Makuuchi, M. , and Aburatani, H. Identification of genes associated with dedifferentiation of hepatocellular carcinoma with expression profiling analysis, Jpn. J. Cancer Res. 93, 636-643 (2002)). However, nothing is known about genes and/or proteins that characterize and/or regulate each differentiation grade of HCC during the course of oncogenesis and development of HCV-associated HCC. Genes and/or proteins that regulate the differentiation grade of HCC can be used for diagnosing the differentiation grade of HCC and for screening anti-cancer agents for the treatment of HCC arising from chronic HCV infection.

Previously, a scoring system for the prediction of cancer recurrence involving the Fisher ratio has been disclosed (WO 03/010337). In addition, comparison of gene expression profiles between hepatitis B virus- and hepatitis C virus-infected hepatocellular carcinoma by oligonucleotide microarray data on the basis of a supervised learning method applying the Fisher ratio has been described (Iizuka et al., Cancer Research 62: 3939-3944 (2002)). Moreover, patterns of gene expression in hematopoietic differentiation have previously been interpreted with self-organizing maps (Tamayo et al., Proc. Natl. Acad. Sci. USA 96: 2907-2912 (1999)). Similarly, a self-organizing map has previously been used for the analysis of yeast gene expression (Törönen et al., FEBS letters 451: 142-146 (1999)).

In the present invention, the inventors describe a method of diagnosing the differentiation grade of tumor and screening anti-cancer agents for the treatment thereof. Particularly, the inventors describe a method of identifying 40 or more genes and/or proteins whose expression correlates with the differentiation grade of HCC, and use of these genes and/or proteins for diagnosing the differentiation grade of HCC and for screening anti-cancer agents for the treatment of HCC in different grades. More particularly, the inventors describe a method of predicting non-cancerous liver, pre-cancerous liver, and each differentiation grade of HCC with 40 genes and/or proteins.

<u>Disclosure of the Invention</u>

Summary of the Invention

The present invention relates to the embodiments characterized in the claims. Thus, it relates to the following items:

1. An in vitro method of defining the differentiation grade of tumor with genes and/or proteins selected by the statistical analyses based on the expression level or pattern of the genes and/or proteins of human tumor tissues obtainable from cancer patients, wherein the genes and/or proteins are selected in descending order of the Fisher ratio and wherein the Fisher ratio is determined without the use of a prior probability, wherein the Fisher ratio for a gene j is given by

$$F(j) = \frac{(\hat{\mu}_j(A) - \hat{\mu}_j(B))^2}{\hat{\sigma}_j^2(A) + \hat{\sigma}_j^2(B)}$$

where $\hat{\mu}_j(i)$ is the sample mean of the expression level of gene j for the samples in Grade i, and $\hat{\sigma}_j^2(i)$ is the sample variance of the expression level of gene $j$ for the samples in Grade $i$.

2. The in vitro method according to item 1, wherein the human tissues are human liver tissues.

3. The in vitro method according to item 2, wherein the differentiation grade of tumor is selected from the group consisting of non-cancerous liver, pre-cancerous liver, well differentiated hepatocellular carcinoma (HCC), moderately differentiated HCC, and poorly differentiated HCC.

4. The in vitro method according to item 3, wherein the genes and/or proteins are differentially expressed between non-cancerous liver and pre-cancerous liver, pre-cancerous liver and well differentiated hepatocellular carcinoma (HCC), well differentiated HCC and moderately differentiated HCC, or moderately differentiated HCC and poorly differentiated HCC.

5. The in vitro method according to any one of items 1 to 4, wherein the expression level or pattern of genes and/or proteins is examined by means of DNA microarray, reverse transcription polymerase-chain reaction or protein array.

6. The in vitro method according to item 5, wherein the number of the genes and/or proteins is between 40 and 100.

7. The in vitro method according to item 5, wherein the number of the genes and/or proteins is between 35 and 45.

8. The in vitro method according to item 7, wherein the number of the genes and/or proteins is 40.

9. An in vitro method of defining the differentiation grade of tumor, the method comprising steps of:

   (a) selecting genes and/or proteins that have the highest Fisher ratios in comparison between non-cancerous liver and pre-cancerous liver, pre-cancerous liver and well differentiated hepatocellular carcinoma (HCC), well differentiated HCC and moderately differentiated HCC, or moderately differentiated HCC and poorly differentiated HCC; and
   (b) defining the differentiation grade of tumor by using the genes and/or proteins, wherein the Fisher ratio is determined without the use of a prior probability, wherein the Fisher ratio for a gene j is given by

$$F(j) = \frac{(\hat{\mu}_j(A) - \hat{\mu}_j(B))^2}{\hat{\sigma}_j^2(A) + \hat{\sigma}_j^2(B)}$$

where $\hat{\mu}_j(i)$ is the sample mean of the expression level of gene $j$ for the samples in Grade $i$, and $\hat{\sigma}_j^2(i)$ is the sample variance of the expression level of gene $j$ for the samples in Grade $i$.

10. An in vitro method of defining the differentiation grade of tumor, the method comprising steps of:

   (a) determining the number of genes and/or proteins to define the differentiation grade of tumor;
   (b) selecting a number of genes and/or proteins decided in step (a) that have the highest Fisher ratios in comparison between non-cancerous liver and pre-cancerous liver, pre-cancerous liver and well differentiated hepatocellular carcinoma (HCC), well differentiated HCC and moderately differentiated HCC, or moderately differentiated HCC and poorly differentiated HCC;
   (c) applying the data of genes and/or proteins selected in step (b) to all samples; and
   (d) defining the differentiation grade of tumor,

wherein the Fisher ratio is determined without the use of a prior probability, wherein the Fisher ratio for a gene j is given by

$$F(j) = \frac{(\hat{\mu}_j(A) - \hat{\mu}_j(B))^2}{\hat{\sigma}_j^2(A) + \hat{\sigma}_j^2(B)}$$

where $\hat{\mu}_j(i)$ is the sample mean of the expression level of gene $j$ for the samples in Grade $i$, and $\hat{\sigma}_j^2(i)$ is the sample variance of the expression level of gene $j$ for the samples in Grade $i$.

11. An in vitro method of defining the differentiation grade of tumor, the method comprising steps of:

(a) determining the number of genes and/or proteins to define the differentiation grade of tumor;

(b) selecting a number of genes and/or proteins decided in step (a) that have the highest Fisher ratios in comparison between non-cancerous liver and pre-cancerous liver, pre-cancerous liver and well differentiated hepatocellular carcinoma (HCC), well differentiated HCC and moderately differentiated HCC, or moderately differentiated HCC and poorly differentiated HCC;

(c) applying the data of genes and/or proteins selected in step (b) to all samples;

(d) designing a minimum distance classifier with the data of genes and/or proteins selected in step (b);

(e) applying the minimum distance classifier designed in step (d) to all samples;

(f) generating self-organizing map with the data of all the genes and/or proteins selected in step (b);

(g) applying the self organizing map generated in step (f) to all samples; and

(h) defining the differentiation grade of tumor,

wherein the Fisher ratio is determined without the use of a prior probability, wherein the Fisher ratio for a gene j is given by

$$F(j) = \frac{(\hat{\mu}_j(A) - \hat{\mu}_j(B))^2}{\hat{\sigma}_j^2(A) + \hat{\sigma}_j^2(B)}$$

where $\hat{\mu}_j(i)$ is the sample mean of the expression level of gene *j* for the samples in Grade *i*, and $\hat{\sigma}_j^2(i)$ is the sample variance of the expression level of gene *j* for the samples in Grade *i*.

Hepatocellular carcinoma (HCC) is one of the most common cancers worldwide. However, there is no therapy that can cure the disease. This is presumably due to sequential changes in characteristics of cancer cells during the development and progression of the disease. Particularly, progression of cancer is often associated with the changes of differentiation grade of tumor cells. Diagnosis and management of such changes of cancer cells will make cancer therapy more effective. In the present invention, genes whose expression correlates with oncogenesis and development of HCC are identified by oligonucleotide microarray representing approximately 11,000 genes from 50 hepatitis C virus (HCV)-associated HCC tissues and 11 non-tumorous (non-cancerous and pre-cancerous) liver tissues.

Differentiation states are divided into 5 grades.

Non-cancerous liver (L0) is the liver that is histologically normal and is seronegative for both hepatitis B virus surface antigen and HCV antibody. Pre-cancerous liver (L1) is the liver that is HCV-infected and is histopathologically diagnosed as chronic hepatitis or liver cirrhosis. Well differentiated HCC (G1) is the HCC consisting of cancer cells that are characterized by an increase in cell density with elevated nuclear/cytoplasm ratios compared to normal hepatocytes but show the morphologies similar to normal hepatocytes. Moderately differentiated HCC (G2) is the HCC consisting of cancer cells that are large and hyperchromatic. There are trabecular- or gland-like structures in cancer cell nest in G2 grade. Poorly differentiated HCC (G3) is the HCC consisting of the cancer cells that are pleomorphic or multinucleate. The tumor grows in solid masses or cell nest devoid of architectural arrangement in G3 grade. G1, G2, and G3 tumors correspond to types I, II, and III of Edmondson & Steiner classification, respectively (Edmondson, H.A. and Steiner, P.E. Primary carcinoma of the liver: a study of 100 cases among 48,900 necropsies, Cancer 7, 462-504 (1954)).

A supervised learning method followed by a random permutation test of oligonucleotide microarray data is used to select genes whose expression significantly changes during the transition from non-cancerous liver without HCV infection (L0) to pre-cancerous liver with HCV infection (L1), from L1 to well differentiated HCC (G1), from G1 to moderately differentiated HCC (G2), and from G2 to poorly differentiated HCC (G3). Self-organizing map with all the selected 40 genes whose expression is significantly altered in each transition stage can correctly predict the differentiation grade of tumor tissues. Thus, these genes can be used for diagnosing the differentiation grade of HCC and for screening anti-cancer agents for the treatment of HCC in each differentiation grade.

Detailed Description of the Invention

In the present invention, human hepatocellular carcinoma (HCC) tissues and non-tumorous (non-cancerous and pre-cancerous) liver tissues are used. HCCs with HCV infection are used for analyzing HCCs. Presence of HCV and/or HBV infection can be determined either by immunoreactivity against anti-HCV antibody and anti-HBV antibody or by amplifying HCV and/or HBV genome by PCR. The differentiation grade of HCC can be determined by histopathological examination, and HCCs are classified into well differentiated HCC (G1), moderately differentiated HCC (G2), and poorly differentiated HCC (G3). Non-tumorous liver samples can be obtained from patients who underwent hepatic resection for benign or metastatic liver tumors. A liver sample without HCV infection is classified as non-cancerous liver (L0), and that with HCV infection is classified as pre-cancerous liver (L1). After resecting liver tissues during surgery, it is preferable that tissues are immediately frozen in liquid nitrogen or acetone containing dry ice and stored at between -70 and -80°C until use. The tissues may or may not be embedded in O.C.T. compound (Sakura-Seiki, Tokyo, Japan, Catalog No. 4583).

The expression of genes and/or proteins of HCC tissues and non-tumorous liver tissues can be analyzed by measuring the level of RNA and/or proteins. In most cases, the level of RNA and/or proteins is determined by measuring fluorescence from substances including fluorescein and rhodamine, chemiluminescence from luminole, radioactivity of radioactive materials including $^3$H, $^{14}$C, $^{35}$S, $^{33}$P $^{32}$P, and $^{125}$I, and optical density. For example, the expression level of RNA and/or proteins is determined by known methods including DNA microarray (Schena, M. et al. Quantitative monitoring of gene expression patterns with a complementary DNA microarray, Science 270, 467-470 (1995) and Lipshutz, R.J. et al. High density synthetic oligonucleotide arrays, Nat. Genet. 21, 20-24 (1999)), RT-PCR (Weis, J.H. et al. Detection of rare mRNAs via quantitative RT-PCR, Trends Genet. 8, 263-264 (1992) and Bustin, S.A. Absolute quantification of mRNA using real-time reverse transcription polymerase chain reaction assays, J. Mol. Endocrinol. 25, 169-193 (2000)), northern blotting and *in situ* hybridization (Parker, R.M. and Barnes, N.M. mRNA: detection in situ and northern hybridization, Methods Mol. Biol. 106, 247-283 (1999)), RNase protection assay (Hod, Y.A. Simplified ribonuclease protection assay, BioTechniques 13, 852-854 (1992) and Saccomanno, C.F. et al. A faster ribonuclease protection assay, Bio-Techniques 13, 846-850 (1992)), western blotting (Towbin, H. et al. Electrophoretic transfer of proteins from polyacrylamide gels to nitrocellulose sheets, Proc. Natl. Acad. Sci. U. S.A. 76, 4350-4354 (1979) and Burnette, W.N. Western blotting: Electrophoretic transfer of proteins form sodium dodecyl sulfate-polyacrylamide gels to unmodified nitrocellulose and radioiodinated protein A, Anal. Biochem. 112, 195-203 (1981)), ELISA assay (Engvall, E. and Perlman, P. Enzyme-linked immunosorbent assay (ELISA) : Quantitative assay of immunoglobulin G, Immunochemistry 8, 871-879 (1971)), and protein array (Merchant, M. and Weinberger, S.R. Review: Recent advancements in surface-enhanced laser desorption/ionization-time of flight-mass spectrometry, Electrophoresis 21, 1164-1177 (2000) and Paweletz, C.P. et al. Rapid protein display profiling of cancer progression directly from human tissue using a protein biochip, Drug Dev. Res. 49, 34-42 (2000)).

Genes and/or proteins that are differently expressed in each differentiation grade of HCC and non-tumorous (non-cancerous and pre-cancerous) liver are selected by comparing the expression level of genes and/or proteins among HCC tissues in each differentiation grade and non-tumorous liver tissues. Genes and/or proteins that are differentially expressed between non-cancerous liver (L0) and pre-cancerous liver that have been infected with HCV (L1) are identified by comparing the expression level of each gene and/or protein between non-cancerous liver tissues and pre-cancerous liver tissues. Genes and/or proteins that are differentially expressed between pre-cancerous liver (L1) and well differentiated HCC (G1) are identified by comparing the expression level of each gene and/or protein between pre-cancerous liver tissues and well differentiated HCC tissues (HCC(G1)). Genes and/or proteins that are differentially expressed between well differentiated HCC (G1) and moderately differentiated HCC (G2) are identified by comparing the expression level of each gene and/or protein between HCC(G1) and moderately differentiated HCC tissues (HCC(G2)). Similarly, genes and/or proteins that are differentially expressed between moderately differentiated HCC (G2) and poorly differentiated HCC (G3) are identified by comparing the expression level of each gene and/or protein between HCC(G2) and poorly differentiated HCC tissues (HCC(G3)).

Differences in the expression level of genes and/or proteins of non-cancerous liver, pre-cancerous liver, well differentiated HCC, moderately differentiated HCC, and poorly differentiated HCC can be analyzed and detected by known methods of statistical analyses. In all experiments for comparing the expression level of genes and/or proteins between two grades selected from L0, L1, G1, G2, and G3, the following procedures are taken.

In the first step, genes and/or proteins with certain expression level (*e.g.* genes with expression level greater than 40 as judged by the arbitrary units by Affymetrix gene chip results) in all the HCC samples and in the non-cancerous and pre-cancerous liver samples are selected. This selection results in certain number of genes and/or proteins. Then, the discriminatory ability of each gene and/or protein to discriminate L0 from L1, L1 from G1, G1 from G2, and G2 from G3 is determined by the Fisher ratio. The Fisher ratio for a gene *j* is given by

$$F(j) = \frac{(\hat{\mu}_j(A) - \hat{\mu}_j(B))^2}{\hat{\sigma}_j^2(A) + \hat{\sigma}_j^2(B)}$$

where $\hat{\mu}_j(i)$ is the sample mean of the expression level of gene *j* for the samples in Grade *i*, and $\hat{\sigma}_j^2(i)$ is the sample variance of the expression level of gene *j* for the samples in Grade *i*.

In the second step, the selected genes and/or proteins are ranked in the order of decreasing magnitude of the Fisher ratio. A random permutation test is also performed to determine the number of genes and/or proteins to define the differentiation grade of HCC. In the permutation test, sample labels are randomly permuted between two grades to be compared, and the Fisher ratio for each gene and/or protein is again computed. This random permutation of sample labels is repeated 1,000 times. The Fisher ratios generated from the actual data are assigned Ps based on the distribution of the Fisher ratios from randomized data. From the distribution of the Fisher ratios based on the randomized data, the genes and/or proteins that are determined to be statistically significant in two grades by the random permutation test are selected. More particularly, the genes and/or proteins that have the P value less than 0.005 by the random permutation test between the two grades are selected. Among these selected genes and/or proteins, 40 genes and/or proteins having the highest Fisher ratios in each comparison between non-cancerous liver (L0) and pre-cancerous liver (L1), pre-cancerous liver (L1) and well differentiated HCC (G1), well differentiated HCC (G1) and moderately differentiated HCC (G2), moderately differentiated HCC (G2) and poorly differentiated HCC (G3) are further selected.

The ability of the selected 40 genes and/or proteins to distinguish non-cancerous liver (L0) from pre-cancerous liver (L1), pre-cancerous liver (L1) from well differentiated HCC (G1), well differentiated HCC (G1) from moderately differentiated HCC (G2), moderately differentiated HCC (G2) from poorly differentiated HCC (G3) is verified by the minimum distance classifier and the self-organizing map (SOM).

The minimum distance classifier is designed using the 40 genes and/or proteins selected in each transition stage. The expression level of each gene and/or protein is normalized to have zero mean and unit variance using all the training samples from two grades. After measuring the Euclidean distance between a sample and each mean vector, the sample is assigned to the grade of the nearest mean vector. The minimum distance classifier that is created with the selected 40 genes and/or proteins in each transition stage is also used to predict the differentiation grade of HCC samples whose differentiation grade is not determined. To diagnose the differentiation grade of HCCs, using $\hat{\mu}_j(A)$ and $\hat{\mu}_j(B)$ previously described, the sample mean $\hat{\mu}_j$ of the mixture consisting of Grades A and B on a gene j is obtained by

$$\hat{\mu}_j = \frac{N_A}{N_A + N_B}\hat{\mu}_j(A) + \frac{N_B}{N_A + N_B}\hat{\mu}_j(B)$$

where $N_i$ is the number of samples from Grade $i$. Next, the sample variance $\hat{\sigma}_j^2$ of the mixture consisting of Grades A and B on the gene $j$ is obtained by

$$\hat{\sigma}_j^2 = \frac{1}{N_A + N_B - 1}\left[(N_A - 1)\hat{\sigma}_j^2(A) + (N_B - 1)\hat{\sigma}_j^2(B) + \frac{N_A N_B}{N_A + N_B}(\hat{\mu}_j(A) - \hat{\mu}_j(B))^2\right]$$

Using $\hat{\mu}_j$ and $\hat{\sigma}_j^2$, $\hat{\mu}$ and $\hat{V}$ are defined by

$$\hat{\mu} = [\hat{\mu}_1, \hat{\mu}_2, \ldots, \hat{\mu}_{40}]^T$$

$$\hat{V} = \begin{bmatrix} \frac{1}{\hat{\sigma}_1} & & & & \mathbf{0} \\ & \frac{1}{\hat{\sigma}_2} & & & \\ & & \ddots & & \\ \mathbf{0} & & & & \frac{1}{\hat{\sigma}_{40}} \end{bmatrix}$$

Then, a sample $x$ is normalized by

$$\tilde{x} = \hat{V}^T (x - \hat{\mu})$$

where $\tilde{x}$ is the normalized sample. Using the normalized samples, the sample mean vector for each grade is obtained. In the minimum distance classifier, the score value is computed by

$$T_1(\tilde{x}) = \| \tilde{x} - \mu_{L0} \|^2 - \| \tilde{x} - \tilde{\mu}_{L1} \|^2$$

$$T_2(\tilde{x}) = \| \tilde{x} - \mu_{L1} \|^2 - \| \tilde{x} - \tilde{\mu}_{G1} \|^2$$

$$T_3(\tilde{x}) = \| \tilde{x} - \mu_{G1} \|^2 - \| \tilde{x} - \tilde{\mu}_{G2} \|^2$$

$$T_4(\tilde{x}) = \| \tilde{x} - \mu_{G2} \|^2 - \| \tilde{x} - \tilde{\mu}_{G3} \|^2$$

Using four minimum distance classifiers, the differentiation grade of HCCs can be diagnosed as follows:

(i) A normalized sample $\tilde{x}$ is classified into Grade L0 if $T_1(\tilde{x})<0$, $T_2(\tilde{x})<0$, $T_3(\tilde{x})<0$ and $T_4(\tilde{x})<0$.
(ii) A normalized sample $\tilde{x}$ is classified into Grade L1 if $T_1(\tilde{x})>0$, $T_2(\tilde{x})<0$, $T_3(\tilde{x})<0$ and $T_4(\tilde{x})<0$.
(iii) A normalized sample $\tilde{x}$ is classified into Grade G1 if $T_1(\tilde{x})>0$, $T_2(\tilde{x})>0$, $T_3(\tilde{x})<0$ and $T_4(\tilde{x})<0$.
(iv) A normalized sample $\tilde{x}$ is classified into Grade G2 if $T_1(\tilde{x})>0$, $T_2(\tilde{x})>0$, $T_3(\tilde{x})>0$ and $T_4(\tilde{x})<0$.
(v) A normalized sample $\tilde{x}$ is classified into Grade G3 if $T_1(\tilde{x})>0$, $T_2(\tilde{x})>0$, $T_3(\tilde{x})>0$ and $T_4(\tilde{x})>0$.

The SOM is a neural network algorithm widely used for clustering and is well known as an efficient tool for the visualization of multidimensional data (Tamayo, P. et al. Interpreting patterns of gene expression with self-organizing maps: methods and application to hematopoietic differentiation, Proc. Natl. Acad. Sci. U.S.A. 96, 2907-2912 (1999) and Sultan, M. et al. Binary tree-structured vector quantization approach to clustering and visualizing microarray data, Bioinformatics Suppl 1, S111-S119 (2002)). The SOM with all the selected 40 genes and/or proteins is carried out according to the method of MATLAB R13 with the SOM toolbox available in the web site, http://www.cis.hut.fi/projects/somtoolbox/ (Kohonen, 2001).

Each set of forty genes and/or proteins whose expression is significantly altered during the transition from non-cancerous liver (L0) to pre-cancerous liver (L1), from pre-cancerous liver (L1) to well differentiated HCC (G1), from well differentiated HCC (G1) to moderately differentiated HCC (G2), from moderately differentiated HCC (G2) to poorly differentiated HCC (G3) is used for diagnosing the grade of hepatocarcinogenesis of HCC, and also for screening anti-cancer agents that are used for the treatment of HCC in each grade.

Each set of forty genes and/or proteins whose expression is significantly altered during the transition from non-cancerous liver (L0) to pre-cancerous liver (L1), from pre-cancerous liver (L1) to well differentiated HCC (G1), from well differentiated HCC (G1) to moderately differentiated HCC (G2), from moderately differentiated HCC (G2) to poorly differentiated HCC (G3) is expressed in bacteria, eukaryotic cells, and cell-free systems. Agents that affect the expression and/or function of the genes and/or proteins are screened by monitoring the expression and/or function. Monoclonal antibodies against the proteins are also raised and used for treating HCC in different grades. As monoclonal antibodies, whole mouse monoclonal antibodies, humanized antibodies, chimeric antibodies, single chain antibodies, divalent single chain antibodies, and/or bi-specific antibodies can be raised against the purified proteins, and they are used for diagnosing the grade of HCC and the treatment thereof.

A kit to examine the expression of the genes and/or proteins is also created. The kit consists of the components including reagents for an RNA extraction, enzymes for synthesis of cDNA and cRNA, DNA chips, oligonucleotide chips, protein chips, probes and primers for the genes, DNA fragments of control genes, and antibodies to the proteins. The components of the kit are easily available from the market.

Brief Description of the Drawings

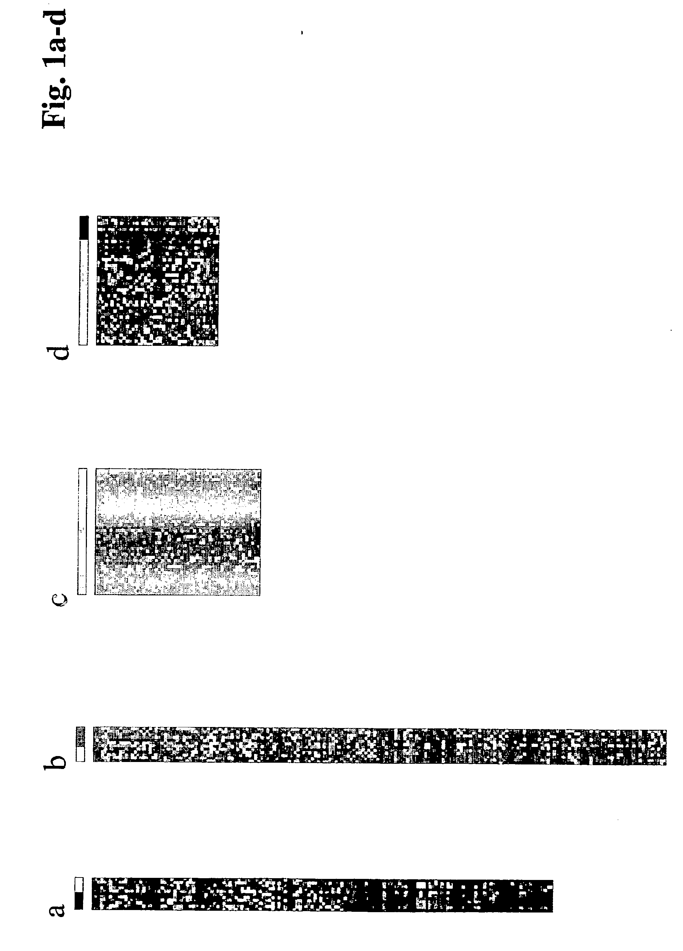

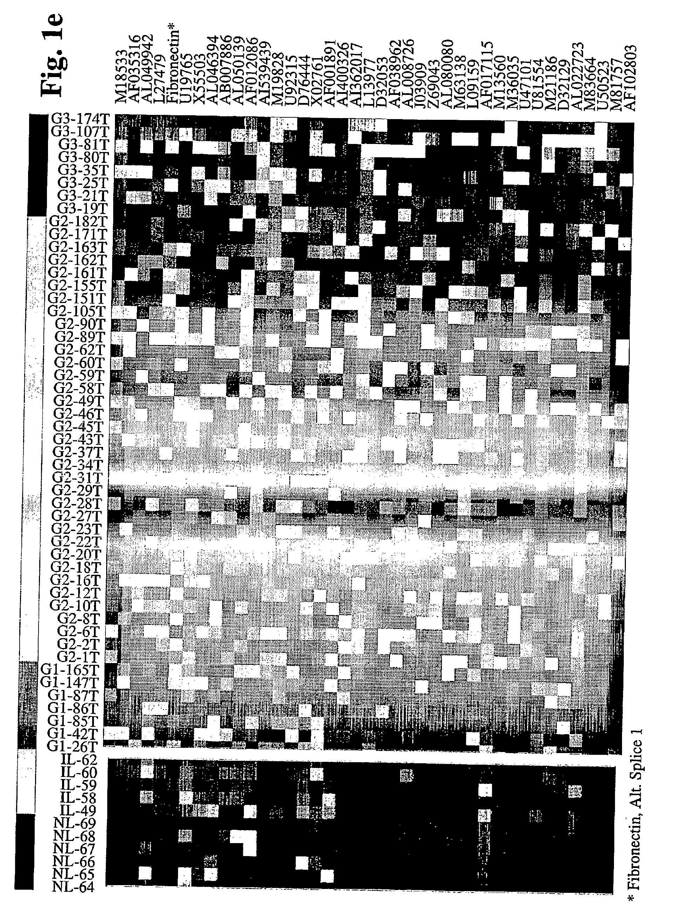

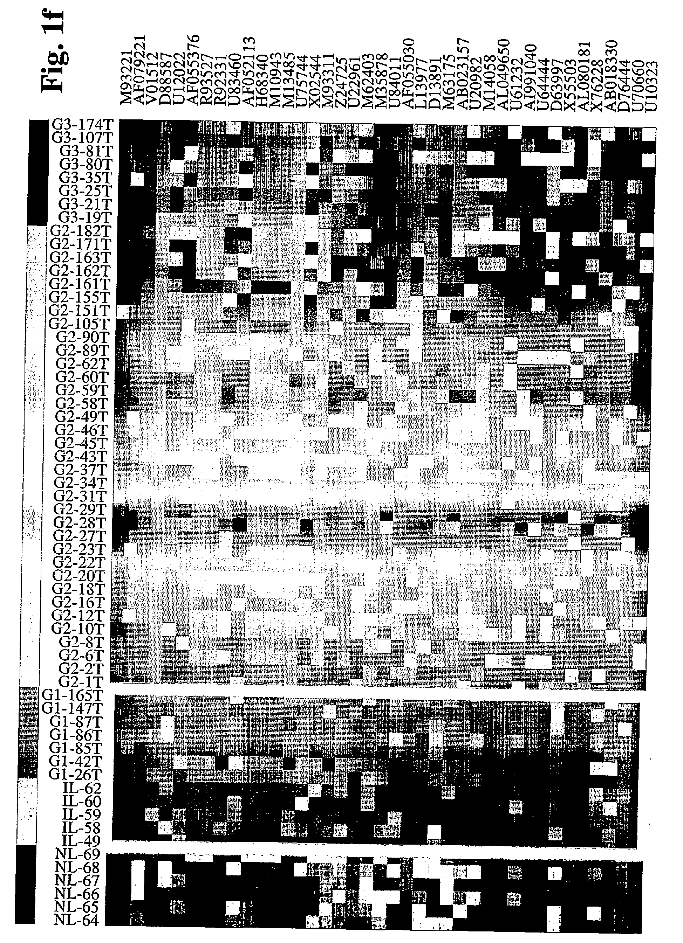

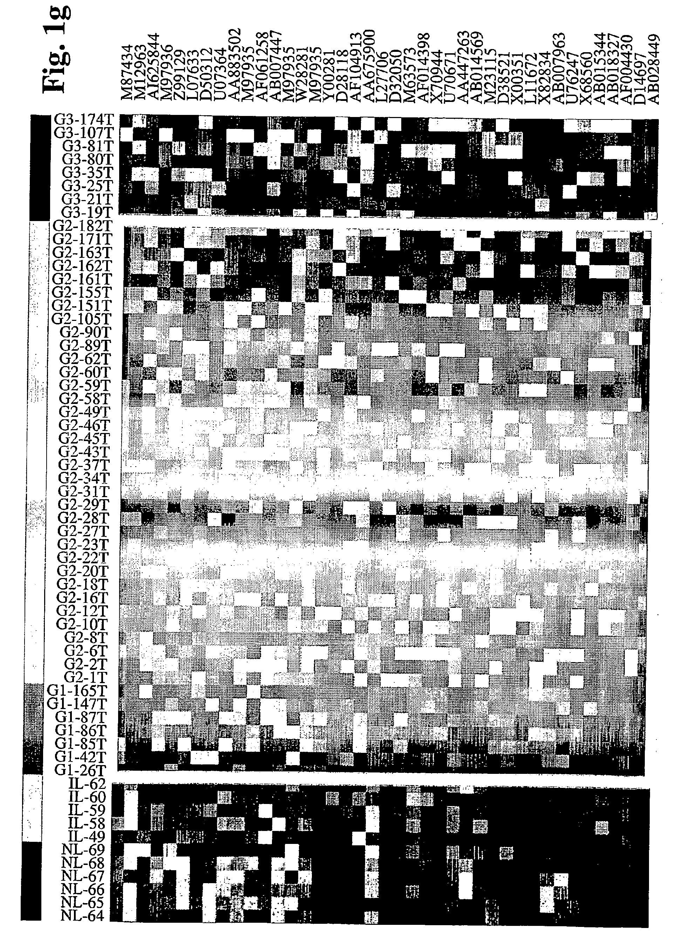

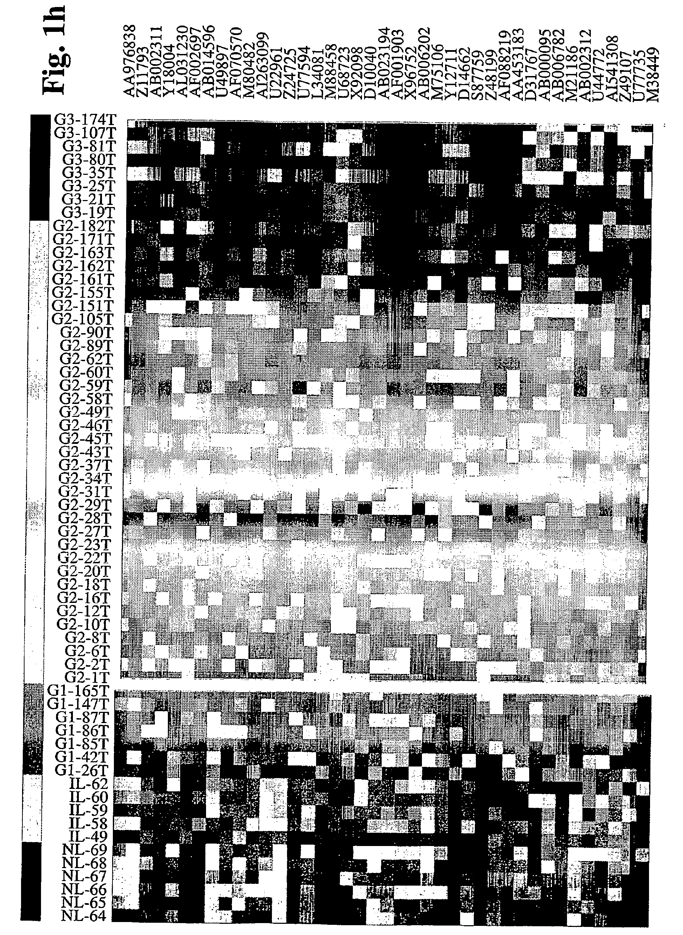

Fig. 1 illustrates color displays of the expression of 152 genes whose expression was significantly altered during the transition from L0 to L1 (a), 191 genes whose expression was significantly altered during the transition from L1 to G1 (b), 54 genes whose expression was significantly altered during the transition from G1 to G2 (c), and 40 genes whose expression was significantly altered during the transition from G2 to G3 (d). Panels e, f, g, and h illustrate expression of the selected 40 genes in each transition stage in all the samples. Expression of the selected 40 genes whose expression was significantly altered during the transition from L0 to L1 (e), from L1 to G1 (f), from G1 to G2 (g), and from G2 to G3 (h) is shown. The selected 40 genes in each transition stage discriminate samples before and after the transition. Genes are shown in decreasing order of the Fisher ratio and are indicated by GenBank accession numbers.

The name of each sample is indicated on top of each photo (e-h) ; NL-64, NL-65, NL-66, NL-67, NL-68, NL-69, IL-49, IL-58, IL-59, IL-60, IL-62, G1-26T, G1-42T, G1-85T, G1-86T, G1-87T, G1-147T, G1-165T, G2-1T, G2-2T, G2-6T, G2-8T, G2-10T, G2-12T, G2-16T, G2-18T, G2-20T, G2-22T, G2-23T, G2-27T, G2-28T, G2-29T, G2-31T, G2-34T, G2-37T, G2-43T, G2-45T, G2-46T, G2-49T, G2-58T, G2-59T, G2-60T, G2-62T, G2-89T, G2-90T, G2-105T, G2-151T, G2-155T, G2-161T, G2-162T, G2-163T, G2-171T, G2-182T, G3-19T, G3-21T, G3-25T, G3-35T, G3-80T, G3-81T, G3-107T, G3-174T, from the left.

The name of each gene is indicated on the right of the photo. In the case of panel e, M18533, AF035316, AL049942, L27479, "Fibronectin, Alt. Splice 1", U19765, X55503, AL046394, AB007886, AL050139, AF012086, AI539439, M19828, U92315, D76444, X02761, AF001891, AI400326, AI362017, L13977, D32053, AF038962, AL008726, J03909, Z69043, AL080080, M63138, L09159, AF017115, M13560, M36035, U47101, U81554, M21186, D32129, AL022723, M83664, U50523, M81757, AF102803, from the top. In the case of panel f, M93221, AF079221, V01512, D88587, U12022, AF055376, R93527, R92331, U83460, AF052113, H68340, M10943, M13485, U75744, X02544, M93311, Z24725, U22961, M62403, M35878, U84011, AF055030, L13977, D13891, M63175, AB023157, U20982, M14058, AL049650, U61232, AI991040, U64444, D63997, X55503, AL080181, X76228, AB018330, D76444, U70660, U10323, from the top. In the case of panel g, M87434, M12963, AI625844, M97936, Z99129, L07633, D50312, U07364, AA883502, M97935, AF061258, AB007447, M97935, W28281, M97935, Y00281, D28118, AF104913, AA675900, L27706, D32050, M63573, AF014398, X70944, U70671, AA447263, AB014569, M23115, D38521, X00351, L11672, X82834, AB007963, U76247, X68560, AB015344, AB018327, AF004430, D14697, AB028449, from the top. In the case of panel h, AA976838, Z11793, AB002311, Y18004, AL031230, AF002697, AB014596, U49897, AF070570, M80482, AI263099, U22961, Z24725, U77594, L34081, M88458, U68723, X92098, D10040, AB023194, AF001903, X96752, AB006202, M75106, Y12711, D14662, S87759, Z48199, AF088219, AA453183, D31767, AB000095, AB006782, M21186, AB002312, U44772, AI541308, Z49107, U77735, M38449, from the top.

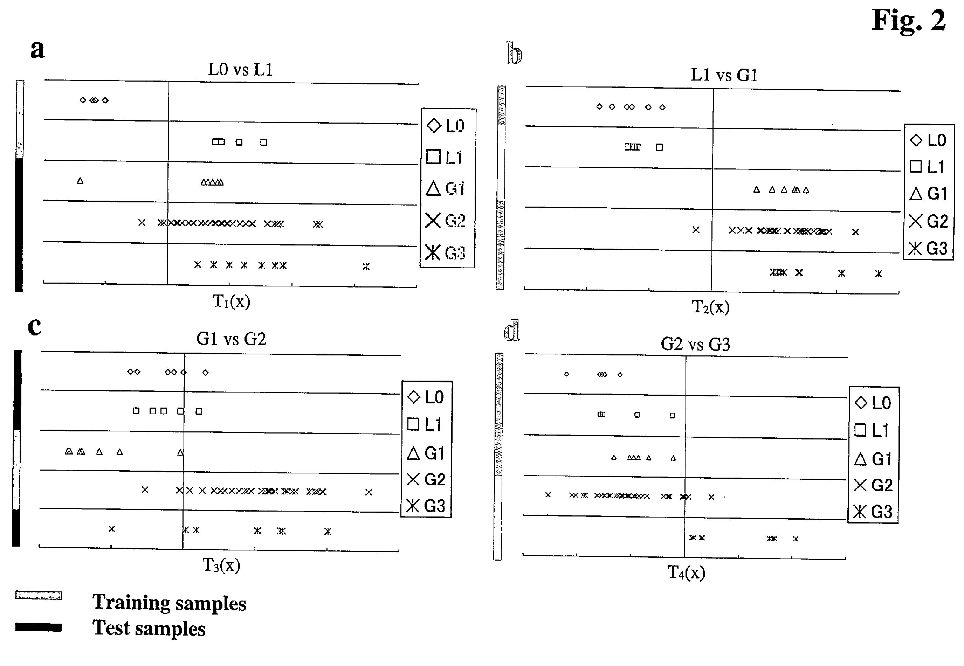

Fig. 2 illustrates the validation of the selected 40 genes in each transition stage to distinguish the differentiation grade of HCC.

In each transition, from L0 to L1 (a), from L1 to G1 (b), From G1 to G2 (c), and from G2 to G3 (d), the minimum distance classifier was constructed with the samples in consecutive two differentiation grades as indicated by the red bar (training samples), and was applied to the samples in the remaining differentiation grades as indicated by the black bar (test samples). The resulting classifier classified the test samples with the accuracy of 92% (a), 98% (b), 84% (c), and 100% (d).

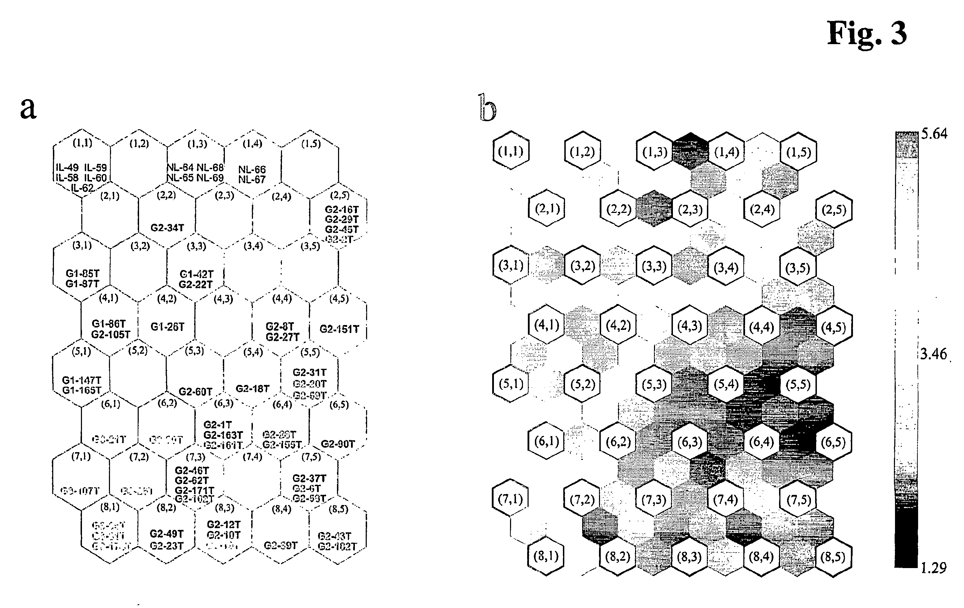

Fig. 3 illustrates the result of analysis by the self-organizing map (SOM) algorithm of the genes whose expression changed during the transition from non-cancerous liver (L0) to pre-cancerous liver (L1), from pre-cancerous liver (L1) to well differentiated HCC (G1), from well differentiated HCC (G1) to moderately differentiated HCC (G2), and from moderately differentiated HCC (G2) to poorly differentiated HCC (G3).

Fig. 3a illustrates clusters of the samples (Table 1). Each cell in the SOM grid corresponds to one cluster. The vectors of neighboring cells are usually located close to each other. (m, n), index of the cell located at m-th row and n-th column. NL-XX, samples from non-cancerous liver without HCV infection (L0) ; IL-XX, samples from HCV-infected pre-cancerous liver (L1) ; G1-XXT, samples from well differentiated HCC (G1) ; G2-XXT, samples from moderately differentiated HCC (G2); G3-XXT, samples from moderately differentiated HCC (G3).

The map shows that the samples clearly formed a sigmoid curve in the order of L0, L1, G1, G2, and G3. G2 samples without vessel involvement (blue letters) are located close to G1 samples and G2 samples with vessel involvement (red letters) are located close to G3 samples.

Fig. 3b illustrates the distance between the neighboring clusters. (m, n), index of the cell located at m-th row and n-th column. The color of the cells indicates the distance between the neighboring clusters; a red color means a long distance. The red cells in the upper area clearly show that the non-tumorous (non-cancerous and pre-cancerous) liver samples and HCC samples are relatively far apart in all the selected 40 genes.

Table 1 illustrates clusters of samples profiled to L0, L1, G1, G2, and G3 as shown in Fig. 3a.

Table 2 illustrates clinicopathologic factors of the HCC used in the present invention.

Table 3 illustrates top-40 discriminatory genes in L0 and L1.

Table 4 illustrates top-40 discriminatory genes in L1 and G1.

Table 5 illustrates top-40 discriminatory genes in G1 and G2.

Table 6 illustrates top-40 discriminatory genes in G2 and G3.

Best Mode for Carrying out the Invention

The following examples merely illustrate the preferred method for identification and use of genes and/or proteins that are differently expressed in non-cancerous liver, pre-cancerous liver, well differentiated HCC, moderately differentiated HCC, and poorly differentiated HCC.

Herein below, the present invention will be specifically described using examples, however, it is not to be construed as being limited thereto.

## Example 1. Preparation of human tissues

Fifty patients underwent surgical treatment for HCC at Yamaguchi University Hospital between May 1997 and August 2000. Written informed consent was obtained from all patients before surgery. The study protocol was approved by the Institutional Review Board for the Use of Human Subjects at the Yamaguchi University School of Medicine. All of the 50 patients were seropositive for HCV antibody (HCVAb) and seronegative for hepatitis B virus surface antigen (HBsAg). A histopathological diagnosis of HCC was made in all cases after surgery. This histopathological examination showed that seven patients had well differentiated HCC (G1), 35 had moderately differentiated HCC (G2), and the remaining eight had poorly differentiated HCC (G3). Clinicopathologic factors were determined according to the International Union against Cancer TNM classification. Fisher's exact test, Student's *t* test, and Mann-Whitney's *U* test were used to elucidate the differences in clinicopathologic characteristics among the 3 grades, G1, G2 and G3 HCC. P<0.05 was considered significant.

Six non-cancerous liver samples were obtained from six patients who underwent hepatic resection for benign or metastatic liver tumors, and confirmed to have histologically normal livers. They were all seronegative for both HBsAg and HCVAb. Five HCV-infected liver samples were also prepared from the non-tumorous areas of five patients with HCC. All five liver samples were histopathologically diagnosed as chronic hepatitis or liver cirrhosis. Informed consent in writing was obtained from all patients before surgery.

## Example 2. Clinicopathologic characteristics of HCCs

Histological examinations showed that, among the 50 HCV-associated HCCs enrolled in this study, seven were well differentiated HCC (G1), 35 were moderately differentiated HCC (G2), and the remaining eight were poorly differentiated HCC (G3) (Table 2). The tumor size of G2 and G3 HCCs was significantly larger than that of G1 HCC (p=0.0007 and p=0.028, respectively, by Mann-Whitney's *U* test). The incidence of vessel involvement in G2 and G3 HCCs was significantly higher than that in G1 HCC (p=0.038 by Fisher's exact test). In parallel to dedifferentiation from G1 to G3, tumor stage was more advanced (p=0.066 by Fisher's exact test). Thus, each type of G1, G2, and G3 HCCs enrolled in this study showed characteristics corresponding to dedifferentiation, *i.e.*, tumor size, metastatic potential, and tumor stage, as proposed by Kojiro (Kojiro, M. Pathological evolution of early hepatocellular carcinoma, *Oncology* **62**, 43-47 (2002)).

## Example 3. Extraction of the RNA from tissues

Pieces of the tissues (about 125 mm$^3$) were suspended in TRIZOL (Life Technologies, Gaithersburg, USA, Catalog No. 15596-018) or Sepasol-RNAI (Nacalai tesque, Kyoto, Japan, Catalog No. 306-55) and homogenized twice with a Polytron (Kinematica, Littau, Switzerland) (5 sec at maximum speed). After addition of chloroform, the tissues homogenates were centrifuged at 15,000 x g for 10 min, and aqueous phases, which contained RNA, were collected. Total cellular RNA was precipitated with isopropyl alcohol, washed once with 70% ethanol, and suspended in DEPC-treated water (Life Technologies, Gaithersburg, USA, Catalog No. 10813-012). After treated with 1.5 units of DNase I (Life Technologies, Gaithersburg, USA, Catalog No. 18068-015), the RNA was re-extracted with TRIZOL/chloroform, precipitated with ethanol, and dissolved in DEPC-treated water. Thereafter, small molecular weight nucleotides were removed by using RNeasy Mini Kit (QIAGEN, Hilden, Germany, Catalog No. 74104) according to a manufacturer's instruction manual. Quality of the total RNA was judged from the ratio of 28S and 18S ribosomal RNA after agarose gel electrophoresis. The purified total RNA was stored at -80 °C in 70% ethanol solution until use.

## Example 4. Synthesis of cDNA and labeled cRNA probes

cDNA was synthesized by using reverse SuperScript Choice System (Life Technologies, Gaithersburg, USA, Catalog No. 18090-019) according to the manufacturer's instruction manual. Five micrograms of the purified total RNA were hybridized with oligo-dT primers (Sawady Technology, Tokyo, Japan) that contained sequences for the T7 promoter and 200 units of SuperScriptII reverse transcriptase and incubated at 42 °C for 1 hr. The resulting cDNA was extracted with phenol/chloroform and purified with Phase Lock Gel® Light (Eppendorf, Hamburg, Germany, Catalog No. 0032 005.101).

cRNA was also synthesized by using MEGAscript T7 kit (Ambion, Austin, USA, Catalog No. 1334) and cDNA as templates according to the manufacturer's instruction. Approximately 5 $\mu$l of the cDNA was incubated with 2 $\mu$l of enzyme mix containing T7 polymerase, 7.5 mM each of adenosine triphosphate (ATP) and guanosine triphosphate (GTP), 5.625 mM each of cytidine triphosphate (CTP) and uridine triphosphate (UTP), and 1.875 mM each of Bio-11-CTP and Bio-16-UTP (ENZO Diagnostics, Farmingdale, USA, Catalog No. 42818 and 42814, respectively) at 37 °C for 6 hr. Mononucleotides and short oligonucleotides were removed by column chromatography on CHROMA SPIN +STE-100 column (CLONTECH, Palo Alto, USA, Catalog No. K1302-2), and the cRNA in the eluates was sedimented by adding ethanol. Quality of the cRNA was judged from the length of the cRNA after agarose gel electrophoresis. The purified cRNA was stored at -80 °C in 70% ethanol solution until use.

## Example 5. Gene expression analysis of HCC in different differentiation grade

Gene expression of human primary tumors from glioma patients was examined by high-density oligonucleotide microarrays (U95A array, Affymetrix, Santa Clara, USA, Catalog No. 510137) (Lipshutz, R.L. et al. High density synthetic oligonucleotide arrays, Nat. Genet. 21, 20-24 (1999)). For hybridization with oligonucleotides on the chips, the cRNA was fragmented at 95 °C for 35 min in a buffer containing 40 mM Tris (Sigma, St. Louis, USA, Catalog No. T1503)-acetic acid (Wako, Osaka, Japan, Catalog No. 017-00256) (pH 8.1), 100 mM potassium acetate (Wako, Osaka, Japan, Catalog No. 160-03175), and 30 mM magnesium acetate (Wako, Osaka, Japan, Catalog No. 130-00095). Hybridization was performed in 200 $\mu$l of a buffer containing 0.1 M 2-(N-Morpholino) ethanesulfonic acid (MES) (Sigma, St. Louis, USA, Catalog No. M-3885) (pH 6.7), 1 M NaCl (Nacalai tesque, Kyoto, Japan, Catalog No. 313-20), 0.01% polyoxylene (10) octylphenyl ether (Wako, Osaka, Japan, Catalog No. 168-11805), 20 $\mu$g herring sperm DNA (Promega, Madison, USA, Catalog No. D181B), 100 $\mu$g acetylated bovine serum albumin (Sigma, St. Louis, USA, Catalog No. B-8894), 10 $\mu$g of the fragmented cRNA, and biotinylated-control oligonucleotides, biotin-5'-CTGAACGGTAGCATCTTGAC-3' (Sawady technology, Tokyo, Japan), at 45 °C for 12 hr. After washing the chips with a buffer containing 0.01 M MES (pH 6.7), 0.1 M NaCl, and 0.001% polyoxylene(10) octylphenyl ether buffer, the chips were incubated with biotinylated anti-streptavidin antibody (Funakoshi, Tokyo, Japan, Catalog No. BA0500) and stained with streptavidin R-Phycoerythrin (Molecular Probes, Eugene, USA, Catalog No. S-866) to increase hybridization signals as described in the instruction manual (Affymetrix, Santa Clara, USA). Each pixel level was collected with laser scanner (Affymetrix, Santa Clara, USA) and levels of the expression of each cDNA and reliability (Present/Absent call) were calculated with Affymetrix GeneChip ver. 3.3 and Affymetrix Microarray Suite ver. 4.0 softwares. From these experiments, expression of approximately 11,000 genes in the human primary tumors of glioma patients was determined.

## Example 6. Statistical analysis of the oligonulceotide microarray data

Genes with average differences greater than 40 (arbitrary units by Affymetrix) in all the 50 HCC samples and the 11 non-tumorous (non-cancerous and pre-cancerous) liver samples were selected. This procedure yielded 3,559 genes out of approximately 11,000. Next, the Fisher ratio was determined (Iizuka, N., Oka, M., Yamada-Okabe, H., Mori, N., Tamesa, T., Okada, T., Takemoto, T. , Tangoku, A., Hamada, K., Nakayama, H., Miyamoto, T., Uchimura, S. , and Hamamoto, Y. Comparison of gene expression profiles between hepatitis B virus- and hepatitis C virus-infected hepatocellular carcinoma by oligonucleotide microarray data based on a supervised learning method, Cancer Res. 62, 3939-3944 (2002) and Luo, J. , Duggan, D.J. , Chen, Y. , Sauvageot, J., Ewing, C.M., Bittner, M.L., Trent, J.M., and Isaacs, W.B. Human prostate cancer and benign prostatic hyperplasia: molecular dissection by gene expression profiling, Cancer Res. 61, 4683-4688 (2001)) to evaluate these genes as discriminators of L0 from L1, L1 from G1, G1 from G2, and G2 from G3. The above 3,559 genes were ranked in the order of decreasing magnitude of the Fisher ratio. A random permutation test was also performed to determine the number of genes to define the differentiation grade of HCC. The random permutation test was carried out as described previously (Iizuka, N., Oka, M., Yamada-Okabe, H., Mori, N., Tamesa, T., Okada, T. , Takemoto, T., Tangoku, A. , Hamada, K., Nakayama, H. , Miyamoto, T., Uchimura, S., and Hamamoto, Y. Comparison of gene expression profiles between hepatitis B virus- and hepatitis C virus-infected hepatocellular carcinoma by oligonucleotide microarray data based on a supervised learning method, Cancer Res. 62, 3939-3944 (2002) and Luo, J., Duggan, D.J., Chen, Y., Sauvageot, J., Ewing, C.M., Bittner, M.L., Trent, J.M., and Isaacs, W.B. Human prostate cancer and benign prostatic hyperplasia: molecular dissection by gene expression profiling, Cancer Res. 61, 4683-4688 (2001)). In the test, sample labels were randomly permuted between two grades to be considered, and the Fisher ratio for each gene was again computed. This random permutation of sample labels was repeated 1,000 times. The Fisher ratios generated from the actual data were then assigned Ps based on the distribution of the Fisher ratios from randomized data. From the distribution of the Fisher ratios based on the randomized data, all of the genes that could pass the random permutation test ($P$< 0.005) were selected. This procedure was performed in all experiments for the comparison of two grades. As a result, 152 genes with the Fisher ratios higher than 4.90 were statistically significant discriminators between L0 and L1. Likewise, 191 genes with the Fisher ratios higher than 4.08 to discriminate L1 from G1, 54 genes with the Fisher ratios higher than 1.52 to discriminate G1 from G2, and 40 genes with the Fisher ratios higher than 1.34 to discriminate G2 from G3, were identified.

**Example 7. Selection of genes whose expression correlates with differentiation grade of HCC**

With oligonucleotide array data, changes in the gene expression during oncogenesis, *i.e.*, from non-cancerous liver (L0) to HCV-infected pre-cancerous liver (L1) and from L1 to well differentiated HCC (G1), and during dedifferentiation of HCC (G1 to G2 and G2 to G3) were analyzed. The supervised learning method followed by a random permutation test identified 152 genes whose expression level was significantly changed during the transition from L0 to L1. Among the 152 genes, 67 were upregulated and 85 were downregulated during this transition. In the same manner, 191 genes whose expression level was significantly changed during the transition from L1 to G1 HCC were identified. Among the 191 genes, 95 were upregulated and 96 were downregulated during this transition. Fifty-four genes appeared to be differentially expressed between G1 and G2 HCCs, and among them the expression of 36 genes was increased and that of 18 genes was decreased during the transition from G1 to G2. Forty genes turned out to be differentially expressed between G2 and G3 HCCs, and among them the expression of 10 genes was increased and that of 30 genes was decreased during the transition from G2 to G3.

To examine performance of the genes selected in each grade in the oncogenesis and development of HCC, the inventors applied data of these genes to all samples. As a result, almost all of these genes selected in each transition stage were placed in L0-L1 transition, L1-G1 transition, G1-G2 transition, and G2-G3 transition. For example, the 191 genes that discriminate L1 from G1 HCC could clearly distinguish non-tumorous livers (L0 and L1) from HCCs (G1, G2, and G3) (Fig. 1). These results indicate that altered level of the selected genes plays central roles in determining each grade of HCC pathogenesis.

**Example 8. Genes whose expression changed during the transition from non-cancerous liver (L0) to pre-cancerous liver (L1)**

Expression of most of immune response-related genes, metabolism-related genes, transport-related genes, proteolysis-related genes, and oncogenesis-related genes was increased, and that of transcription-related genes was decreased during the transition from L0 to L1 (Table 3).

Immune response-related genes include MHC class I family (HLA-A, -C, -E, and -F), MHC class II family (HLA-DPB1 and HLA-DRA), CD74, NK4, LILRB1, FCGR3B, and IFI30. Upregulation of an interferon (IFN) inducible gene such as IFI30 may represent host defense against viral infection; however, it should be noted that several IFN-related genes were decreased during dedifferentiation of G1 to G2 as mentioned in the following section (see Example 10).

Metabolism-related genes include KARS, ALDOA, ASAH, MPI, and GAPD. Increased levels of KARS and ALDOA enhance protein biosynthesis and glycolysis, respectively. Upregulaton of ASAH, MPI, and GAPD augments biosynthesis of fatty acid, mannose, and glyceraldehyde, respectively.

Transport-related genes include VDAC3, SSR4, BZRP, and ATOX1. SSR4 is responsible for the effective transport of newly synthesized polypeptides. ATOX1 is a copper transporter and an increase in its expression causes activation of various metabolic pathways, because many enzymes require copper ion as a cofactor of enzymatic activity.

Proteolysis-related genes include CST3 and CTSD. CST3 is involved in vascular formation. Increased serum level of CTSD protein was observed in cirrhotic patients who may develop pre-cancerous hepatic nodules (Leto, G., Tumminello, F.M., Pizzolanti, G., Montalto, G., Soresi, M., Ruggeri, I., and Gebbia, N. Cathepsin D serum mass concentrations in patients with hepatocellular carcinoma and/or liver cirrhosis, Eur. J. Clin. Chem. Clin. Biochem. 34, 555-560 (1996)).

Oncogenesis-related genes include MBD2, RPS19, RPS3, RPS15 , and RPS12. DNA methylation is a common epigenetic change in many malignancies, thus, DNA methylation patterns are determined by the enzymatic processes of methylation and demethylation. Upregulation of MBD2, which inhibits transcription from methylated DNA, plays an important role in downregulation of tumor suppressor genes carrying methylated DNA at their promoter regions.

Downregulation of a transcription-related gene, RB1CC1, was observed during the transition from L0 to L1. The RB1CC1 protein is a major regulator of the tumor suppressor gene RB1, thereby decreased levels of RB1CC1 can promote oncogenesis via decreased activity of RB1 protein.

Thus, HCV-infected pre-cancerous liver is characterized by the altered expression of these genes, which suggests that initiation of hepatocarcinogenesis occurs during HCV infection. Among genes whose expression changes during the transition from L0 to L1, those involved in proteolysis and oncogenesis may serve as molecular targets for chemoprevention of HCV-associated HCC.

**Example 9. Genes whose expression changed during the transition from pre-cancerous liver (L1) to well differentiated HCC (G1)**

Genes whose expression was altered during the transition from L1 to G1 include most oncogenesis-related genes, signal transduction-related genes, transcription-related genes, transport-related genes, detoxification-related genes, and immune response-related genes (Table 4).

Oncogenesis-related genes such as BNIP3L, FOS, MAF, and IGFBP3 that can induce apoptosis of some cancer cells and IGFBP4 that acts as an inhibitor of IGF-induced cell proliferation were downregulated during the transition, indicating downregulation of these genes is also important for the promotion of hepatocarcinogenesis. Previous report also showed the decreased expression of IGFBP3 and IGFBP4 in HCC compared with non-tumorous liver (Okabe, H., Satoh, S., Kato, T., Kitahara, O., Yanagawa, R., Yamaoka, Y., Tsunoda, T., Furukawa, Y., and Nakamura, Y. Genomewide analysis of gene expression in human hepatocellular carcinomas using cDNA microarray: identification of genes involved in viral carcinogenesis and tumor progression, Cancer Res. 61, 2129-2137 (2001) and Delpuech, O., Trabut, J.B., Carnot, F., Feuillard, J., Brechot, C., and Kremsdorf, D. Identification, using cDNA macroarray analysis, of distinct gene expression profiles associated with pathological and virological features of hepatocellular carcinoma, Oncogene 21, 2926-2937 (2002)). The data of the present invention provide additional insights that downregulation of these two genes has already occurred in well differentiated HCC. MAF functions as a regulator for cell differentiation. BNIP3L induces cell apoptosis via inhibiting activity of BCL2. In some cases, expression of FOS seems to be associated with apoptotic cell death. Thus, downregulation of these five genes is likely to trigger the transformation of hepatocyte after chronic HCV infection.

Signal transduction-related genes such as CAMKK2, GMFB, RALBP1, CDIPT, ZNF259, and RAC1, and transcription-related genes such as DRAP1, ILF2, BMI1, and PMF1 were upregulated during the transition from L1 to G1. Other signal transduction-related genes such as CALM1, RAB14, TYROBP, and MAP2K1 were downregulated during this transition. Downregulation of TYROBP in G1 HCC may reflect decreased immune response. Alteration of the expression of genes involved in various signal transduction pathways may reflect a true portrait in well differentiated HCC arising from HCV-infected pre-cancerous liver.

Transport-related genes such as TBCE, ATP6V1E, ATOX1, and SEC61G were upregulated, and those such as SLC31A1 and DDX19 were downregulated during the transition from L1 to G1. ATOX1 that is an intracellular copper transporter was upregulated during the transition from L0 to L1, and it was further upregulated during the transition from L1 to G1. Since an excessive copper is toxic or even lethal to the hepatocytes, distinct expression of ATOX1 genes alters intracellular copper ion concentrations, thereby promotes DNA damage and cell injury. In fact, a recent study showed the preventive effect of copper-chelating agents on tumor development in the murine HCC xenograft model (Yoshii, J., Yoshij i, H., Kuriyama, S., Ikenaka, Y., Noguchi, R., Okuda, H., Tsujinoue, H., Nakatani, T., Kishida, H., Nakae, D., Gomez, D.E., De Lorenzo, M.S., Tejera, A.M., and Fukui, H. The copper-chelating agent, trientine, suppresses tumor development and angiogenesis in the murine hepatocellular carcinoma cells, Int. J. Cancer. 94, 768-773 (2001)).

DNA damage and cell injury can be augmented by the downregulation of an antioxidant gene CAT and detoxification-related genes such as MT1H, MT1E, MT1F, MT1B, MT3, and UGT2B7, promoting the dedifferentiation of HCC.

Using anti-hyaluronan receptor-1 antibody, Carreira et al. showed that the number of lymphatic vessels was smaller in HCC than in non-tumorous liver tissues such as liver cirrhosis (Mouta Carreira, C., Nasser, S.M., di Tomaso, E., Padera, T.P., Boucher, Y., Tomarev, S.I., and Jain, R.K. LYVE-1 is not restricted to the lymph vessels: expression in normal liver blood sinusoids and down-regulation in human liver cancer and cirrhosis, Cancer Res. 61, 8079-8084 (2001)). In the present invention, expression of immune response-related genes such as ORM1, C1R, C6, IL4R, C8B, and C1S was decreased during the transition from L1 to G1, indicating that changes in microenvironment in HCC occur during the transition from L1 to G1. As reported previously, many genes encoding complement component were downregulated during this transition (Okabe, H., Satoh, S., Kato, T., Kitahara, O., Yanagawa, R., Yamaoka, Y., Tsunoda, T., Furukawa, Y., and Nakamura, Y. Genome-wide analysis of gene expression in human hepatocellular carcinomas using cDNA microarray: identification of genes involved in viral carcinogenesis and tumor progression, Cancer Res. 61, 2129-2137 (2001) and Iizuka, N., Oka, M., Yamada-Okabe, H., Mori, N., Tamesa, T., Okada, T., Takemoto, T., Tangoku, A., Hamada, K., Nakayama, H., Miyamoto, T., Uchimura, S., and Hamamoto, Y. Comparison of gene expression profiles between hepatitis B virus- and hepatitis C virus-infected hepatocellular carcinoma by oligonucleotide microarray data based on a supervised learning method, Cancer Res. 62, 3939-3944 (2002)).

**Example 10. Genes whose expression changed during the transition from well differentiated HCC (G1) to moderately differentiated HCC (G2)**

Genes whose expression was altered during the transition from G1 to G2 include IFN-related genes, cell structure and motility-related genes, transcription-related genes, and tumor suppressor genes (Table 5).

During transition from G1 to G2, the most prominent genetic changes appeared to be downregulation of IFN-related genes such as OAS2, STAT1, PSME1, ISGF3G, and PSMB9. Similar genetic changes were also observed in prostate cancer cells (Shou, J., Soriano, R., Hayward, S.W., Cunha, G.R., Williams, P.M., and Gao, W.Q. Expression profiling of a human cell line model of prostatic cancer reveals a direct involvement of interferon signaling in prostate tumor progression, Proc. Natl. Acad. Sci. U.S.A. 99, 2830-2835 (2002)). IFN acts not only as an antiviral agent but also as an anticancer agent; however, certain types of HCC do not respond to IFN. Downregulation of the IFN-related genes can attenuate response of tumor cells to IFN, suggesting that resistance of HCC to IFN is exploited during the transition from G1 to G2. Among the IFN-related genes, STAT1 appeared four times in our list of discriminators of G1 from G2 (Table 5). Unlike other genes of the same family, STAT1 functions as a tumor suppressor (Bromberg, J.F. Activation of STAT proteins and growth control, Bioessays 23, 161-169 (2001)). Interestingly, IFN treatment increases STAT1 expression in hepatocyte as well as many IFN-related genes (Radaeva, S., Jaruga, B., Hong, F., Kim, W.H., Fan, S., Cai, H., Strom, S., Liu, Y., El-Assal, O., and Gao, B. Interferon-alpha activates multiple STAT signals and down-regulates c-Met in primary human hepatocytes, Gastroenterology 122, 1020-1034 (2002)). Upregulation of STAT1 in HCC cell lines was observed during differentiation induced by sodium butyrate (Hung, W.C. and Chuang, L.Y. Sodium butyrate enhances STAT 1 expression in PLC/PRF/5 hepatoma cells and augments their responsiveness to interferon-alpha, Br. J. Cancer 80, 705-710 (1999)). The facts that STAT1 is a transcriptional target of the IGF-independent apoptotic effect of IGFBP3 (Spagnoli, A., Torello, M., Nagalla, S.R., Horton, W.A., Pattee, P., Hwa, V., Chiarelli, F., Roberts, C.T. Jr., and Rosenfeld, R.G. Identification of STAT-1 as a molecular target of IGFBP-3 in the process of chondrogenesis, J. Biol. Chem. 277, 18860-18867 (2002)) and that IGFBP3 is downregulated during the transition from L1 to G1 strongly suggest that decreased expression of STAT1 during the transition from G1 to G2 HCC facilitate the further dedifferentiation of HCC.

Transcription-related gene TRIM16 that is involved in a variety of biological processes including cell growth, differentiation, and pathogenesis, and tumor suppressor gene TPD52L2 that promotes cell proliferation were also upregulated during the transition from G1 to G2. Upregulation of these genes in G2 HCC may promote growth and invasion of tumor cells.

**Example 11. Genes whose expression changed during the transition from moderately differentiated HCC (G2) to poorly differentiated HCC (G3)**

Genes whose expression was altered during the transition from G2 to G3 include proteolysis-related genes, BCL2-related gene, and metabolism- and energy generation-related genes (Table 6).

SPINT1 and LGALS9 turned out to be upregulated during the transition from G2 to G3. SPINT1 is involved in regulation of proteolytic activation of hepatocyte growth factor (HGF) in injured tissues. Previously, Nagata *et al.* showed that transduction of antisense SPINT1 (HAI-1) inhibited the growth of human hepatoma cells, suggesting that SPINT1 plays an important role in the progression of HCC (Nagata, K., Hirono, S., Ido, A., Kataoka, H., Moriuchi, A., Shimomura, T., Hori, T., Hayashi, K., Koono, M., Kitamura, N., and Tsubouchi, H. Expression of hepatocyte growth factor activator and hepatocyte growth factor activator inhibitor type 1 in human hepatocellular carcinoma, Biochem. Biophys. Res. Commun. 289, 205-211 (2001)). LGALS9 belongs to a lectin family that is involved in cell adhesion, cell growth regulation, inflammation, immunomodulation, apoptosis, and metastasis. Several galectins are thought to be related to cancer cell adhesion (Ohannesian, D.W., Lotan, D., Thomas, P., Jessup, J.M., Fukuda, M., Gabius, H.J., and Lotan, R. Carcinoembryonic antigen and other glycoconjugates act as ligands for galectin-3 in human colon carcinoma cells, Cancer Res. 55, 2191-2199 (1995)).

BNIP3, a BCL2-related gene, was downregulated during the transition from G2 to G3. BNIP3 shares 56% amino acid sequence identity with BNIP3L. As mentioned above, expression of BNIP3L was decreased during the transition from L1 to G1. Because BCL2 functions as an anti-apoptotic factor, downregulation of BNIP3L and BNIP3 promotes oncogenesis, facilitating the dedifferentiation of tumor cells.

Many metabolism- and energy generation- related genes were also downregulated during this transition. In addition, expression of PGRMC1 encoding a liver-rich protein that binds to progesterone and RARRES2 was also decreased during the transition from G2 to G3. Decreased expression of RARRES2 may be the causative of poor response of G3 HCC to retinoic acids.

**Example 12. Color display of the expression of the selected genes in each transition stage**

Expression of 152 genes whose expression was significantly altered during the transition from L0 to L1 (Fig. 1a), 191 genes whose expression was significantly altered during the transition from L1 to G1 (Fig. 1b), 54 genes whose expression was significantly altered during the transition from G1 to G2 (Fig. 1c), and 40 genes whose expression was significantly altered during the transition from G2 to G3 (Fig. 1d) was shown by color display. These genes clearly distinguished the samples in the two consecutive differentiation grades. Fig. 1e-h indicate the expression of the selected 40 genes in each transition stage in all the samples. Expression of the selected 40 genes whose expression was significantly altered during the transition from L0 to L1 (Fig. 1e), from L1 to G1 (Fig. If), from G1 to G2 (Fig. 1g), and from G2 to G3 (Fig. 1h) was also shown by color display. The selected 40 genes in each transition stage discriminated samples before and after the transition.

**Example 13. Validation of the selected 40 genes in each transition stage to distinguish the differentiation grade of HCC**

To validate discriminative performance of the selected 40 genes in each transition stage, the minimum distance classifier with the selected 40 genes in each transition stage was created. In each transition, the minimum distance classifier was constructed with the samples in consecutive two differentiation grades as indicated by the red bar (training samples), and was applied to the samples in the remaining differentiation grades as indicated by the black bar (test samples) (Fig. 2). The resulting classifier classified the test samples with the accuracy of 92% (Fig. 2a), 98% (Fig. 2b), 84% (Fig. 2c), and 100% (Fig. 2d).

**Example 14. Analysis by the self-organizing map (SOM) algorithm of the genes whose expression changed during the transition from non-cancerous liver (L0) to pre-cancerous liver (L1), from pre-cancerous liver (L1) to well differentiated HCC (G1), from well differentiated HCC (G1) to moderately differentiated HCC (G2), and from moderately differentiated HCC (G2) to poorly differentiated HCC (G3)**

Expression of the genes whose expression was statistically significantly different between non-cancerous liver (L0) and pre-cancerous liver (L1), pre-cancerous liver (L1) and well differentiated HCC (G1), well differentiated HCC (G1) and moderately differentiated HCC (G2), moderately differentiated HCC (G2) and poorly differentiated HCC (G3) was analysed according to the method of MATLAB R13 with the SOM toolbox available in the web site, http://www.cis.hut.fi/projects/somtoolbox/ (Kohonen, 2001). 40 genes in each comparison between non-cancerous liver (L0) and pre-cancerous liver (L1), pre-cancerous liver (L1) and well differentiated HCC (G1), well differentiated HCC (G1) and moderately differentiated HCC (G2), moderately differentiated HCC (G2) and poorly differentiated HCC (G3) were used. The vectors of neighboring cells were located close to each other in the 155-dimential gene space (Fig. 3a), where (m, n) indicated the cell located at m-th row and n-th column, NL-XX indicated samples from non-cancerous liver without HCV infection (L0), IL-XX indicated samples from HCV-infected pre-cancerous liver (L1) G1-XXT indicated samples from well differentiated HCC (G1), G2-XXT indicated samples from moderately differentiated HCC (G2), G3-XXT indicated samples from moderately differentiated HCC (G3). The map showed that the samples clearly formed a sigmoid curve in the order of L0, L1, G1, G2, and G3. G2 samples without vessel involvement (blue letters) were located close to G1 samples and G2 samples with vessel involvement (red letters) were located close to G3 samples (Fig. 3a). G2 samples without venous invasion were located close to G1 samples and G2 samples with venous invasion were located close to G3 samples. Thus, the SOM classified G2 samples into two subtypes, *i.e.*, tumor with venous invasion and that without venous invasion, in the stream of dedifferentiation grade. When the distance between the neighboring clusters was shown by colors where red indicated long distance, the red cells in the upper area clearly demonstrated that the non-tumorous (non-cancerous and pre-cancerous) liver and HCC samples were relatively far apart in the 155-dimentional genes space (Fig. 3b).

Industrial Applicability

Hepatocellular carcinoma (HCC) is one of the most common cancers worldwide. However, there is no therapy that can cure the disease. This is presumably due to sequential changes in characteristics of cancer cells during the development and progression of the disease. Particularly, progression of cancer is often associated with the changes of differentiation grade of tumor cells. Diagnosis and management of such changes of cancer cells will make cancer therapy more effective. In the present invention, genes whose expression correlates with oncogenesis and development of HCC are identified. A supervised learning method followed by a random permutation test is used to select genes whose expression significantly changes during the transition from non-cancerous liver without HCV infection (L0) to pre-cancerous liver with HCV infection (L1), from L1 to well differentiated HCC (G1), from G1 to moderately differentiated HCC (G2), and from G2 to poorly differentiated HCC (G3). The minimum distance classifier and the self-organizing map (SOM) with the selected 40 genes whose expression is significantly altered in each transition stage can correctly predict the differentiation grade of tumor tissues. Thus, these genes can be used for diagnosing the differentiation grade of HCC and for screening anti-cancer agents for the treatment of HCCs in each differentiation grade.

Table 1. Clusters of samples profiled to L0, L1, G1, G2, and G3.

| cell | sample |
|---|---|
| (1,1) | IL-49, IL-58, IL-59, IL-60, IL-62 |
| (1,2) | |
| (1,3) | NL-64, NL-65, NL-68, NL-69 |
| (1,4) | NL-66, NL-67 |
| (1,5) | |
| (2,1) | |
| (2,2) | G2-34T |
| (2,3) | |
| (2,4) | |
| (2,5) | G2-16T, G2-29T, G2-45T |
| | G2-2T |
| (3,1) | G1-85T, G1-87T |
| (3,2) | |
| (3,3) | G1-42T |
| | G2-22T |
| (3,4) | |
| (3,5) | |
| (4,1) | G1-86T |
| | G2-105T |
| (4,2) | G1-26T |
| (4,3) | |
| (4,4) | G2-8T, G2-27T |
| (4,5) | G2-151T |
| (5,1) | G1-147T, G1-165T |
| (5,2) | |
| (5,3) | G2-60T |
| (5,4) | G2-18T |
| (5,5) | G2-31T |
| | G2-20T, G2-59T |
| (6,1) | G3-21T |
| (6,2) | G3-80T |
| (6,3) | G2-1T, G2-163T |
| | G2-161T |
| (6,4) | G2-28T, G2-155T |
| (6,5) | G2-90T |
| (7,1) | G3-107T |
| (7,2) | G3-25T |

(continued)

| cell | sample |
|------|--------|
| (7,3) | G2-46T, G2-62T, G2-171T |
| | G2-162T |
| (7,4) | |
| (7,5) | G2-37T |
| | G2-6T, G2-58T |
| (8,1) | G3-35T, G3-81T, G3-174T |
| (8,2) | G2-49T |
| | G2-23T |
| (8,3) | G2-12T |
| | G2-10T |
| | G3-19T |
| (8,4) | G2-89T |
| (8,5) | G2-43T, G2-182T |

Table 2. Clinicopathologic characteristics per study group.

| Factors | Well (G1) | Moderately (G2) | Poorly (G3) | P value |
|---------|-----------|-----------------|-------------|---------|
| Sex | | | | N.S. |
|   Male | 4 | 24 | 6 | |
|   Female | 3 | 11 | 2 | |
| Age (year) | 65.3±2.6 | 65.4±1.2 | 67.2±3.3 | N.S. |
| Primary lesion | | | | N.S. |
|   Single tumor | 6 | 15 | 2 | |
|   Multiple tumors | 1 | 20 | 6 | |
| Tumor size (cm) | 2.0±0.3 | 5.0±0.5 | 6.0±2.5 | p=0.0007 (G1 vs G2)<br><br>p=0.028 (G1 vs G3) |
| Stage* | | | | p=0.066 |
|   I | 6 | 10 | 2 | |
|   II | 1 | 17 | 3 | |
|   IIIA/IV | 0 | 8 | 3 | |
| Venous invasion* | | | | p=0.038 |
|   (-) | 7 | 21 | 3 | |
|   (+) | 0 | 14 | 5 | |
| Non-tumorous liver | | | | N.S. |
|   Normal or chronic hepatitis | 2 | 15 | 2 | |
|   Liver cirrhosis | 5 | 20 | 6 | |
| *, Tumor differentiation, stage, and venous invasion were determined on the basis of TNM classification of UICC. Fisher's exact test, Student's *t* test, and Mann-Whitney's *U* test were used to elucidate the differences in backgrounds between each differentiation grade.<br>N.S., not significant. | | | | |

Table 3. Top-40 discriminatory genes in L0 and L1.

| Eighteen genes downregulated in L1 in comparison with L0 | | | | | |
|---|---|---|---|---|---|
| Fisher ratio | GB number | Description | Symbol | Locus | Function |
| 50.45 | M18533 | dystrophin | DMD | Xp21.2 | cytoskeleton |
| 23.02 | AF035316 | homolog to tubulin beta chain | | 6p24.3 | unknown |
| 20.65 | AL049942 | zinc finger protein 337 | ZNF337 | 20p11.1 | unknown |
| 18.34 | L27479 | Friedreich ataxia region gene X123 | X123 | 9q13-q21 | unknown |
| 16.63 | Fibronectin Alt. Splice 1 | fibronectin (Alt. Splice 1) | | | extracellular matirx |
| 16.13 | U19765 | zinc finger protein 9 | ZNF9 | 3q21 | transcription/ retroviral nucleic acid binding protein |
| 14.91 | X55503 | metallothionein IV | MTIV | 16q13 | detoxification |
| 13.71 | AL046394 | poly (rC) binding protein 3 | PCBP3 | 21q22.3 | RNA-binding protein/ post-transcription al control |
| 12.56 | AB007886 | KIAA0426 gene product | KIAA0426 | 6p22.2-p21.3 | unknown |
| 12.41 | AL050139 | hypothetical protein FLJ13910 | FLJ13910 | 2p11.1 | unknown |
| 12.37 | AF012086 | RAN binding protein 2-like 1 | RANBP2L1 | 2q12.3 | signal transduction/ small GTP-binding protein |
| 11.66 | AI539439 | S100 calcium binding protein A2 | S100A2 | 1q21 | extracellular stimuli and cellular responses |
| 11.24 | M19828 | apolipoprotein B | APOB | 2p24-p23 | lipid metabolism |
| 10.59 | U92315 | sulfotransferase family, cytosolic, 2B, member 1 | SULT2B1 | 19q13.3 | steroid metabolism |
| 10.53 | D76444 | zinc finger protein 103 homolog (mouse) | ZFP103 | 2p11.2 | central nervous system development |
| 10.50 | X02761 | fibronectin 1 | FN1 | 2q34 | extracellular matirx/ cell adhesion and motility |
| 10.20 | AF001891 | zinc finger protein-like 1 | ZFPL1 | 11q13 | unknown |
| 9.74 | AI400326 | EST | | 2 | UniGene Cluster Hs. 356456 |
| Twenty-two genes upregulated in L1 in comparison with L0 | | | | | |
| Fisher ratio | GB number | Description | Symbol | Locus | Function |
| 40.49 | AI362017 | cystatin C | CST3 | 20p11.21 | cysteine protease inhibitor |

(continued)

| Twenty-two genes upregulated in L1 in comparison with L0 | | | | | |
|---|---|---|---|---|---|
| Fisher ratio | GB number | Description | Symbol | Locus | Function |
| 21.66 | L13977 | prolylcarboxypeptidase (angiotensinase C) | PRCP | 11q14 | metabolism/ lysosome-related protein |
| 20.59 | D32053 | lysyl-tRNA synthetase | KARS | 16q23-q24 | protein biosynthesis |
| 13.70 | AF038962 | voltage-dependent anion channel 3 | VDAC3 | 8p11.2 | transport of adenine nucleotides |
| 11.90 | AL008726 | protective protein for beta-galactosidase (cathepsin A) | PPGB | 20q13.1 | lysosomal protein/ enzyme activator |
| 11.71 | J03909 | interferon, gamma-inducible protein 30 | IFI30 | 19p13.1 | lysosomal thiol reductase/IFN-inducible |
| 11.32 | Z69043 | signal sequence receptor, delta | SSR4 | Xq28 | translocatation of newly synthesized polypeptides |
| 11.17 | AL080080 | thioredoxin-related transmembrane protein | TXNDC | 14q21.3 | redox reaction |
| 11.15 | M63138 | cathepsin D | CTSD | 11p15.5 | lysosomal aspartyl protease/proteolysis |
| 11.12 | L09159 | ras homolog gene family, member A | ARHA | 3p21.3 | oncogenesis/actin cytoskeleton reorganization |
| 10.99 | AF017115 | cytochrome c oxidase subunit IV isoform 1 | COX4I1 | 16q22-qter | energy pathway |
| 10.76 | M13560 | CD74 antigen | CD74 | 5q32 | immune response |
| 10.22 | M36035 | benzodiazapine receptor | BZRP | 22q13.31 | flow of cholesterol into mitochondria |
| 10.08 | U47101 | nitrogen fixation cluster-like | NIFU | 12q24.1 | unknown |
| 9.70 | U81554 | calcium/calmodulin-dependent protein kinase II gamma | CAMK2G | 10q22 | signal transduction |
| 9.59 | M21186 | cytochrome b-245, alpha polypeptide | CYBA | 16q24 | energy generation |
| 9.47 | D32129 | major histocompatibility complex, class I, A | HLA-A | 6p21.3 | immune response |
| 9.44 | AL022723 | major histocompatibility complex, class I, F | HLA-F | 6p21.3 | immune response |
| 9.41 | M83664 | major histocompatibility complex, class II, DP beta 1 | HLA-DPB1 | 6p21.3 | immune response |
| 9.16 | U50523 | actin related protein 2/3 complex, subunit 2 | ARPC2 | 13q12-q13 | cell motility and cytoskeleton |

(continued)

| Twenty-two genes upregulated in L1 in comparison with L0 | | | | | |
|---|---|---|---|---|---|
| Fisher ratio | GB number | Description | Symbol | Locus | Function |
| 9.02 | M81757 | ribosomal protein S19 | RPS19 | 19q13.2 | oncogenesis/RNA-binding protein |
| 8.89 | AF102803 | catenin (cadherin-associated protein), alpha 1 | CTNNA1 | 5q31 | cell adhesion |

Table 4. Top-40 discriminatory genes in L1 and G1.

| Twenty-eight genes downregulated in G1 in comparison with L1 | | | | | |
|---|---|---|---|---|---|
| Fisher ratio | GB number | Description | Symbol | Locus | Function |
| 26.84 | M93221 | mannose receptor, C type 1 | MRC1 | 10p13 | phagocytosis and pinocytosis |
| 26.08 | AF079221 | BCL2/adenovirus E1B 19kD interacting protein 3-like | BNIP3L | 8p21 | tumor suppressor/ induction of apoptosis |
| 21.46 | V01512 | v-fos FBJ murine osteosarcoma viral oncogene homolog | FOS | 14q24.3 | oncogenesis/ transcription |
| 21.45 | D88587 | ficolin 3 (Hakata antigen) | FCN3 | 1p35.3 | extracellular space |
| 20.15 | U12022 | calmodulin 1 | CALM1 | 14q24-q31 | signal transduction/ calcium-binding protein |
| 19.73 | AF055376 | v-maf musculoaponeurotic fibrosarcoma oncogene homolog | MAF | 16q22-q23 | oncogenesis/ transcription |
| 19.19 | R93527 | metallothionein 1H | MT1H | 16q13 | detoxification |
| 18.19 | R92331 | metallothionein 1E | MT1E | 16q13 | detoxification |
| 17.65 | U83460 | solute carrier family 31, member1 | SLC31A1 | 9q31-q32 | copper ion transport |
| 17.30 | AF052113 | RAB14, member RAS oncogene family | RAB14 | 9q32-q34.11 | Ras superfamily member of GTP-binding proteins |
| 15.26 | H68340 | RNA helicase-related protein | RNAHP | 17q22 | alteration of RNA secondary structure |
| 14.96 | M10943 | metallothionein 1F | MT1F | 16q13 | detoxification |
| 14.18 | M13485 | metallothionein 1B | MT1B | 16q13 | detoxification |
| 13.34 | U75744 | deoxyribonuclease l-like 3 | DNASE1L3 | 3p21.1-3p14.3 | DNA metabolism |
| 12.65 | X02544 | orosomucoid 1 | ORM1 | 9q31-q32 | immune response/ acute-phase response |
| 11.95 | M93311 | metallothionein 3 | MT3 | 16q13 | detoxification |
| 11.58 | Z24725 | mitogen inducible 2 | MIG2 | 14q22.1 | cell cycle and cell proliferation |
| 11.52 | U22961 | unknown | | | unknown |

(continued)

| Twenty-eight genes downregulated in G1 in comparison with L1 | | | | | |
|---|---|---|---|---|---|
| Fisher ratio | GB number | Description | Symbol | Locus | Function |
| 11.45 | M62403 | insulin-like growth factor binding protein 4 | IGFBP4 | 17q12-q21.1 | signal transduction/cell proliferation |
| 11.01 | M35878 | insulin-like growth factor binding protein 3 | IGFBP3 | 7p13-p12 | signal transduction/cell proliferation |
| 10.80 | U84011 | amylo-1, 6-glucosidase, 4-alpha-glucanotransferase | AGL | 1p21 | glycogen degradation |
| 10.74 | AF055030 | PHD zinc finger protein XAP135, isoform b | XAP135 | 6q27 | unknown |
| 10.29 | L13977 | prolylcarboxypeptidase (angiotensinase C) | PRCP | 11q14 | metabolism/lysosome-related protein |
| 10.02 | D13891 | inhibitor of DNA binding 2 | ID2 | 2p25 | negative regulator of cell differentiation |
| 9.95 | M63175 | autocrine motility factor receptor | AMFR | 16q21 | signal transduction/cell motility |
| 9.94 | AB023157 | KIAA0940 protein | KIAA0940 | 10q23.33 | unknown |
| 9.76 | U20982 | insulin-like growth factor binding protein 4 | IGFBP4 | 17q12-q21.1 | signal transduction/cell proliferation |
| 9.09 | M14058 | complement component 1, r subcomponent | C1R | 12p13 | immune response |
| Twelve genes upregulated in G1 in comparison with L1 | | | | | |
| Fisher ratio | GB number | Description | Symbol | Locus | Function |
| 30.42 | AL049650 | small nuclear ribonucleoprotein polypeptides B and B1 | SNRPB | 20p13 | RNA processing/ modification/ RNA splicing |
| 20.95 | U61232 | tubulin-specific chaperone e | TBCE | 1q42.3 | microtubule/ cochaperonin |
| 11.95 | AI991040 | DR1-associated protein 1 | DRAP1 | 11q13.3 | transcription |
| 10.96 | U64444 | ubiquitin fusion degradation 1-like | UFD1L | 22q11.21 | proteolysis |
| 10.71 | D63997 | golgi autoantigen, golgin subfamily a, 3 | GOLGA3 | 12q24.33 | stabilization of Golgi structure |
| 10.60 | X55503 | metallothionein IV | MT4 | 16q13 | detoxification |
| 10.23 | AL080181 | Immunoglobulin superfamily, member 4 | IGSF4 | 11q23.2 | It possess low similarity to viral receptor |
| 10.01 | X76228 | ATPase, $H^+$ transporting, lysosomal 31kD, V1 subunit E | ATP6V1E | 22q11.1 | proton transport |
| 9.77 | AB018330 | calcium/calmodulin-dependent protein kinase kinase 2, beta | CAMKK2 | 12q24.2 | signal transduction/ calcium-binding protein |

(continued)

| Twelve genes upregulated in G1 in comparison with L1 | | | | | |
|---|---|---|---|---|---|
| Fisher ratio | GB number | Description | Symbol | Locus | Function |
| 9.41 | D76444 | zinc finger protein 103 homolog (mouse) | ZFP103 | 2p11.2 | central nervous system development |
| 9.31 | U70660 | ATX1 antioxidant protein 1 homolog (yeast) | ATOX1 | 5q32 | copper homeostasis and ion transport |
| 9.10 | U10323 | interleukin enhancer binding factor 2, 45kD | ILF2 | 1q21.1 | transcription |

Table 5. Top-40 discriminatory genes in G1 and G2.

| Fifteen genes downregulated in G2 in comparison with G1 | | | | | |
|---|---|---|---|---|---|
| Fisher ratio | GB number | Description | Symbol | Locus | Function |
| 2.89 | M87434 | 2'-5'-oligoadenylate synthetase 2 | OAS2 | 12q24.2 | antiviral response protein/IFN-inducible |
| 2.63 | M12963 | class I alcohol dehydrogenase alpha subunit | ADH1A | 4q21-q23 | detoxification |
| 2.51 | AI625844 | hypothetical protein FLJ20378 | | | unknown |
| 2.43 | M97936 | signal transducer and activator of transcription 1 | STAT1 | 2q32.2 | transcription/ IFN-signaling pathway |
| 2.12 | Z99129 | heat shock transcription factor 2 | HSF2 | 6q22.33 | transcription |
| 2.08 | L07633 | proteasome activator subunit1 | PSME1 | 14q11.2 | proteolysis and peptidolysis/IFN-inducible |
| 2.06 | D50312 | potassium inwardly-rectifying channel subfamily J, member8 | KCNJ8 | 12p11.23 | potassium transport |
| 2.02 | U07364 | proteasome activator subunit1 | PSME1 | 14q11.2 | proteolysis and peptidolysis/IFN-inducible |
| 2 | AA883502 | ubiquitin-conjugating enzyme E2L6 | UBE2L6 | 11q12 | proteolysis and peptidolysis |
| 1.85 | M97935 | signal transducer and activator of transcription 1 | STAT1 | 2q32.2 | transcription/ transcription/ IFN-signaling pathway |
| 1.83 | AF061258 | LIM protein | LIM | 4q22 | signal transduction |
| 1.74 | AB007447 | FLN 29 gene product | FLN29 | 12q | signal transduction |
| 1.72 | M97935 | signal transducer and activator of transcription 1 | STAT1 | 2q32.2 | transcription/ IFN-signaling pathway |

(continued)

| Fifteen genes downregulated in G2 in comparison with G1 | | | | | |
|---|---|---|---|---|---|
| Fisher ratio | GB number | Description | Symbol | Locus | Function |
| 1.7 | W28281 | GABA (A) receptor-associated protein like 1 | GABARAPL 1 | 12p13.1 | microtubule associated protein |
| 1.66 | M97935 | signal transducer and activator of transcription 1 | STAT1 | 2q32. 2 | transcription/ IFN-signaling pathway |
| Twenty-five genes upregulated in G2 in comparison with G1 | | | | | |
| Fisher ratio | GB number | Description | Symbol | Locus | Function |
| 4.41 | Y00281 | ribophorin I | RPNI | 3q21.3-q25.2 | protein modification/RNA binding |
| 3.25 | D28118 | zinc finger protein 161 | ZNF161 | 17q23.3 | transcription |
| 2.83 | AF104913 | eukaryotic protein synthesis initiation factor 4 gamma | EIF4G1 | 3q27-qter | tanslation |
| 2.27 | AA675900 | formin 3 binding protein 3 | FNBP3 | 2q23.3 | proteolysis and peptidolysis |
| 2.27 | L27706 | chaperonin containing TCP1, subunit 6A (zeta 1) | CCT6A | 7p14.1 | chaperone/protein folding |
| 2.15 | D32050 | alanyl-tRNA synthetase | AARS | 16q22 | tRNA processing/ protein synthesis |
| 2.1 | M63573 | peptidylprolyl isomerase B | PPIB | 15q21-q22 | chaperone/immune response |
| 2.09 | AF014398 | inositol(myo)-1(or 4)-monophosphatase 2 | IMPA2 | 18p11.2 | signal transduction |
| 2.08 | X70944 | splicing factor proline/ glutamine rich | SFPQ | 1p34.2 | mRNA splicing/ mRNA processing |
| 2.03 | U70671 | ataxin 2 related protein | A2LP | 7 | unknown |
| 1.89 | AA447263 | golgi reassembly stacking protein 2, 55kDa | GORASP2 | 2p24.3-q21.3 | golgi stacking |
| 1.87 | AB014569 | KIAA0669 gene product | KIAA0669 | 3 | unknown |
| 1.85 | M23115 | ATPase, Ca$^{++}$ transporting, cardiac muscle, slow twitch 2 | ATP2A2 | 12q23-q24.1 | small molecule transport |
| 1.83 | D38521 | proteasome activator 200 kDa | PA200 | 2p16.2 | proteolysis and peptidolysis |
| 1.82 | X00351 | actin, beta | ACTB | 7p15-p12 | cytoskeleton |
| 1.75 | L11672 | zinc finger protein 91 | ZNF91 | 19p13.1-p12 | transcription |
| 1.75 | X82834 | golgi autoantigen, golgin subfamily a, 4 | GOLGA4 | 3p22-p21.3 | vesicle transport |
| 1.74 | AB007963 | KIAA0494 gene product | KIAA0494 | 1pter-p22.1 | unknown |

(continued)

| Twenty-five genes upregulated in G2 in comparison with G1 | | | | | |
|---|---|---|---|---|---|
| Fisher ratio | GB number | Description | Symbol | Locus | Function |
| 1.74 | U76247 | seven in absentia homolog 1 (Drosophila) | SIAH1 | 16q12 | proteolysis and peptidolysis/apoptosis |
| 1.73 | X68560 | Sp3 transcription factor | SP3 | 2q31 | transcription |
| 1.73 | AB015344 | ubiquilin 2 | UBQLN2 | Xp11.23-p11.1 | ubiquitination |
| 1.73 | AB018327 | activity-dependent neuroprotector | ADNP | 20q13.13-q13.2 | unknown |
| 1.7 | AF004430 | tumor protein D52-like 2 | TPD52L2 | 20q13.2-q13.3 | cell proliferation |
| 1.67 | D14697 | farnesyl diphosphate synthase | FDPS | 1q21.2 | cholesterol biosynthesis |
| 1.67 | AB028449 | Dicer1, Dcr-1 homolog (Drosophila) | DICER1 | 14q32.2 | RNA helicase |

Table 6. Top-40 discriminatory genes in G2 and G3.

| Thirty genes downregulated in G3 in comparison with G2 | | | | | |
|---|---|---|---|---|---|
| Fisher ratio | GB number | Description | Symbol | Locus | Function |
| 2.36 | AA976838 | apolipoprotein C-I | APOC1 | 19q13.2 | lipid metabolism |
| 2.20 | Z11793 | selenoprotein P, plasma, 1 | SEPP1 | 5q31 | antioxidant activity |
| 1.86 | AB002311 | PDZ domain containing guanine nucleotide exchange factor 1 | PDZ-GEF1 | 4q32.1 | Ras/Rap1A-associating signal transduction |
| 1.80 | Y18004 | sex comb on midleg-like 2 (Drosophila) | SCML2 | Xp22 | transcription/ embryogenesis and morphogenesis |
| 1.76 | AL031230 | aldehyde dehydrogenase 5 family, member A1 | ALDH5A1 | 6p22 | electron transporter/ aminobutyrate catabolism |
| 1.71 | AF002697 | BCL2/adenovirus E1B 19kD interacting protein 3 | BNIP3 | 14q11.2-q12 | apoptosis |
| 1.65 | AB014596 | F-box and WD-40 domain protein 1B | FBXW1B | 5q35.1 | ubiquitination |
| 1.64 | U49897 | phenylalanine hydroxylase | PAH | 12q22-q24.2 | amino acid biosynthesis |
| 1.62 | AF070570 | Homo sapiens clone 24473 mRNA sequence | | 4 | unknown |
| 1.59 | M80482 | paired basic amino acid cleaving system 4 | PACE4 | 15q26 | cell-cell signalling/ proteolysis |
| 1.59 | AI263099 | FLJ31305 fis or clone LIVER1000104 | | 16 | similar to Rattus norvegicus kidney-specific protein mRNA |
| 1.57 | U22961 | unknown | | | unknown |
| 1.57 | Z24725 | mitogen inducible 2 | MIG2 | 14q22.1 | cell cycle control |

(continued)

| Thirty genes downregulated in G3 in comparison with G2 | | | | | |
|---|---|---|---|---|---|
| Fisher ratio | GB number | Description | Symbol | Locus | Function |
| 1.53 | U77594 | retinoic acid receptor responder (tazarotene induced) 2 | RARRES2 | 7q35 | retinoic acid receptor/ retinoic acid-inducble |
| 1.49 | L34081 | bile acid Coenzyme A: amino acid N-acyltransferase | BAAT | 9q22.3 | liver enzyme for glycine and bile acid metabolisms |
| 1.49 | M88458 | KDEL endoplasmic reticulum protein retention receptor 2 | KDELR2 | 7p22.2 | intracellular protein traffic |
| 1.48 | U68723 | checkpoint suppressor 1 | CHES1 | 14q24.3-q31 | transcription/cell cycle |
| 1.48 | X92098 | coated vesicle membrane protein | RNP24 | 12q24.31 | intracellular protein traffic |
| 1.44 | D10040 | fatty-acid-Coenzyme A ligase, long-chain 2 | FACL2 | 4q34-q35 | fatty acid metabolism |
| 1.43 | AB023194 | KIAA0977 protein | KIAA0977 | 2q24.3 | unknown |
| 1.42 | AF001903 | L-3-hydroxyacyl-Coenzyme A dehydrogenase, short chain | HADHSC | 4q22-q26 | mitochondrial enzyme/ energy generation |
| 1.40 | X96752 | L-3-hydroxyacyl-Coenzyme A short dehydrogenase, short chain | HADHSC | 4q22-q26 | mitochondrial generation enzyme/energy generation |
| 1.40 | AB006202 | succinate dehydrogenasecomplex, subunit D | SDHD | 11q23 | mitochondrial protein/ electron transporter |
| 1.37 | M75106 | carboxypeptidase B2 | CPB2 | 13q14.11 | proteolysis and peptidolysis peptidolysis |
| 1.37 | Y12711 | rogesterone receptor membrane component 1 | PGRMC1 | Xq22-q24 | liver-rich protein that binds to progesterone |
| 1.36 | D14662 | anti-oxidant protein 2 | AOP2 | 1q23.3 | antioxidant activity/non-selenium glutathione peroxidase |
| 1.36 | S87759 | protein phosphatase 1A | PPM1A | 14q23.1 | cellular stress responses |
| 1.36 | Z48199 | syndecan 1 | SDC1 | 2p24.1 | cell adhesion and metastasis |
| 1.35 | AF088219 | chemokine (C-C motif) ligand 14 | CCL14 | 17q11.2 | cell proliferation |
| 1.35 | AA453183 | EST | | | unknown |
| Ten genes upregulated in G3 in comparison with G2 | | | | | |
| Fisher GB | ratio | number Description | Symbol | Locus | Function |
| 2.80 | D31767 | DAZ associated protein 2 | DAZAP2 | 2q33-q34 | RNA-binding protein |

(continued)

| Ten genes upregulated in G3 in comparison with G2 | | | | | |
|---|---|---|---|---|---|
| Fisher GB | ratio | number Description | Symbol | Locus | Function |
| 2.57 | AB000095 | serine protease inhibitor, Kunitz type 1 | SPINT1 | 15q13.3 | inhibitor specific for HGFactivator |
| 2.40 | AB006782 | galectin 9 | LGALS9 | 17q11.1 | cell adhesion and metastasis |
| 2.18 | M21186 | cytochrome b-245, alpha polypeptide | CYBA | 16q24 | energy generation |
| 1.96 | AB002312 | bromodomain adjacent to zinc finger domain 2A | BAZ2A | 12q24.3-qter | DNA-binding protein |
| 1.84 | U44772 | palmitoyl-protein thioesterase 1 | PPT1 | 1p32 | neuronal maturation |
| 1.77 | AI541308 | S100 calcium binding protein A13 | S100A13 | 1q21 | extracellular stimuli and cellular responses |
| 1.53 | Z49107 | galectin 9 | LGALS9 | 17q11.1 | cell adhesion and metastasis |
| 1.36 | U77735 | pim-2 oncogene | PIM2 | Xp11.23 | cell proliferation |
| 1.34 | M38449 | transforming growth factor, beta 1 | TGFB1 | 19q13.2 | cell growth and adhesion |

**Claims**

1. An in vitro method of defining the differentiation grade of tumor with genes and/or proteins selected by the statistical analyses based on the expression level or pattern of the genes and/or proteins of human tumor tissues obtainable from cancer patients, wherein the genes and/or proteins are selected in descending order of the Fisher ratio and wherein the Fisher ratio is determined without the use of a prior probability, wherein the Fisher ratio for a gene j is given by

$$F(j) = \frac{(\hat{\mu}_j(A) - \hat{\mu}_j(B))^2}{\hat{\sigma}_j^2(A) + \hat{\sigma}_j^2(B)}$$

where $\hat{\mu}_j(i)$ is the sample mean of the expression level of gene $j$ for the samples in Grade $i$, and $\hat{\sigma}_j^2(i)$ is the sample variance of the expression level of gene $j$ for the samples in Grade $i$.

2. The in vitro method according to claim 1, wherein the human tissues are human liver tissues.

3. The in vitro method according to claim 2, wherein the differentiation grade of tumor is selected from the group consisting of non-cancerous liver, pre-cancerous liver, well differentiated hepatocellular carcinoma (HCC), moderately differentiated HCC, and poorly differentiated HCC.

4. The in vitro method according to claim 3, wherein the genes and/or proteins are differentially expressed between non-cancerous liver and pre-cancerous liver, pre-cancerous liver and well differentiated hepatocellular carcinoma (HCC), well differentiated HCC and moderately differentiated HCC, or moderately differentiated HCC and poorly differentiated HCC.

5. The in vitro method according to any one of claims 1 to 4, wherein the expression level or pattern of genes and/or proteins is examined by means of DNA microarray, reverse transcription polymerase-chain reaction or protein array.

6. The in vitro method according to claim 5, wherein the number of the genes and/or proteins is between 40 and 100.

7. The in vitro method according to claim 5, wherein the number of the genes and/or proteins is between 35 and 45.

8. The in vitro method according to claim 7, wherein the number of the genes and/or proteins is 40.

9. An in vitro method of defining the differentiation grade of tumor, the method comprising steps of:

(a) selecting genes and/or proteins that have the highest Fisher ratios in comparison between non-cancerous liver and pre-cancerous liver, pre-cancerous liver and well differentiated hepatocellular carcinoma (HCC), well differentiated HCC and moderately differentiated HCC, or moderately differentiated HCC and poorly differentiated HCC; and
(b) defining the differentiation grade of tumor by using the genes and/or proteins, wherein the Fisher ratio is determined without the use of a prior probability, wherein the Fisher ratio for a gene j is given by

$$F(j) = \frac{(\hat{\mu}_j(A) - \hat{\mu}_j(B))^2}{\hat{\sigma}_j^2(A) + \hat{\sigma}_j^2(B)}$$

where $\hat{\mu}_j(i)$ is the sample mean of the expression level of gene $j$ for the samples in Grade $i$, and $\hat{\sigma}_j^2(i)$ is the sample variance of the expression level of gene $j$ for the samples in Grade $i$.

10. An in vitro method of defining the differentiation grade of tumor, the method comprising steps of:

(a) determining the number of genes and/or proteins to define the differentiation grade of tumor;
(b) selecting a number of genes and/or proteins decided in step (a) that have the highest Fisher ratios in comparison between non-cancerous liver and pre-cancerous liver, pre-cancerous liver and well differentiated hepatocellular carcinoma (HCC), well differentiated HCC and moderately differentiated HCC, or moderately differentiated HCC and poorly differentiated HCC;
(c) applying the data of genes and/or proteins selected in step (b) to all samples; and
(d) defining the differentiation grade of tumor,

wherein the Fisher ratio is determined without the use of a prior probability, wherein the Fisher ratio for a gene j is given by

$$F(j) = \frac{(\hat{\mu}_j(A) - \hat{\mu}_j(B))^2}{\hat{\sigma}_j^2(A) + \hat{\sigma}_j^2(B)}$$

where $\hat{\mu}_j(i)$ is the sample mean of the expression level of gene $j$ for the samples in Grade $i$, and $\hat{\sigma}_j^2(i)$ is the sample variance of the expression level of gene $j$ for the samples in Grade $i$.

11. An in vitro method of defining the differentiation grade of tumor, the method comprising steps of:

(a) determining the number of genes and/or proteins to define the differentiation grade of tumor;
(b) selecting a number of genes and/or proteins decided in step (a) that have the highest Fisher ratios in comparison between non-cancerous liver and pre-cancerous liver, pre-cancerous liver and well differentiated hepatocellular carcinoma (HCC), well differentiated HCC and moderately differentiated HCC, or moderately differentiated HCC and poorly differentiated HCC;
(c) applying the data of genes and/or proteins selected in step (b) to all samples;
(d) designing a minimum distance classifier with the data of genes and/or proteins selected in step (b);
(e) applying the minimum distance classifier designed in step (d) to all samples;
(f) generating self-organizing map with the data of all the genes and/or proteins selected in step (b);
(g) applying the self organizing map generated in step (f) to all samples; and
(h) defining the differentiation grade of tumor,

wherein the Fisher ratio is determined without the use of a prior probability, wherein the Fisher ratio for a gene j is given by

$$F(j) = \frac{(\hat{\mu}_j(A) - \hat{\mu}_j(B))^2}{\hat{\sigma}_j^2(A) + \hat{\sigma}_j^2(B)}$$

where $\hat{\mu}_j(i)$ is the sample mean of the expression level of gene $j$ for the samples in Grade $i$, and $\hat{\sigma}_j^2(i)$ is the sample variance of the expression level of gene $j$ for the samples in Grade $i$.

**Patentansprüche**

1. In vitro-Verfahren zur Bestimmung des Differenzierungsgrads eines Tumors mit Genen und/oder Proteinen, die ausgewählt sind durch die statistischen Analysen basierend auf dem Expressionsspiegel oder -muster der Gene und/oder Proteine von menschlichen Tumorgeweben, die von Krebspatienten erhältlich sind, wobei die Gene und/oder Proteine in absteigender Reihenfolge des Fisher-Verhältnisses ausgewählt sind und wobei das Fisher-Verhältnis ohne die Verwendung einer vorherigen Wahrscheinlichkeitsaussage bestimmt wird, wobei das Fisher-Verhältnis für ein Gen j gegeben ist durch

$$F(j) = \frac{(\hat{\mu}_j(A) - \hat{\mu}_j(B))^2}{\hat{\sigma}_j^2(A) + \hat{\sigma}_j^2(B)}$$

wobei $\hat{\mu}_j(i)$ der Proben-Mittelwert des Expressionsspiegels des Gens $j$ für die Proben des Grades $i$ ist, und $\hat{\sigma}_j^2(i)$ die Proben-Abweichung des Expressionsspiegels des Gens $j$ für die Proben des Grades $i$ ist.

2. In vitro-Verfahren nach Anspruch 1, wobei die menschlichen Gewebe menschliche Lebergewebe sind.

3. In vitro-Verfahren nach Anspruch 2, wobei der Differenzierungsgrad von Tumoren ausgewählt ist aus der Gruppe bestehend aus nicht-kanzeröse Leber, prä-kanzeröse Leber, gut differenziertes Leberkarzinom (HCC), gemäßigt differenziertes HCC und schlecht differenziertes HCC.

4. In vitro-Verfahren nach Anspruch 3, wobei die Gene und/oder Proteine differentiell exprimiert sind zwischen nicht-kanzeröser Leber und prä-kanzeröser Leber, prä-kanzeröser Leber und gut differenziertem Leberkarzinom (HCC), gut differenziertem HCC und gemäßigt differenziertem HCC, oder gemäßigt differenziertem HCC und schlecht differenziertem HCC.

5. In vitro-Verfahren nach einem der Ansprüche 1 bis 4, wobei der Expressionsspiegel oder das Expressionsmuster der Gene und/oder Proteine untersucht wird durch DNA-Mikroarray, reverse Transkriptions-Polymerase-Kettenreaktion oder Proteinarray.

6. In vitro-Verfahren nach Anspruch 5, wobei die Zahl der Gene und/oder Proteine zwischen 40 und 100 ist.

7. In vitro-Verfahren nach Anspruch 5, wobei die Zahl der Gene und/oder Proteine zwischen 35 und 45 ist.

8. In vitro-Verfahren nach Anspruch 7, wobei die Zahl der Gene und/oder Proteine 40 ist.

9. In vitro-Verfahren zur Definition des Differenzierungsgrads von Tumoren, wobei das Verfahren die Schritte umfasst:

(a) das Auswählen von Genen und/oder Proteinen, die die höchsten Fisher-Verhältnisse haben im Vergleich zwischen nicht-kanzeröser Leber und prä-kanzeröser Leber, prä-kanzeröser Leber und gut differenziertem Leberkarzinom (HCC), gut differenziertem HCC und gemäßigt differenziertem HCC, oder gemäßigt differenziertem HCC und schlecht differenziertem HCC; und
(b) das Definieren des Differenzierungsgrads von Tumoren durch Verwendung der Gene und/oder Proteine,

wobei das Fisher-Verhältnis ohne die Verwendung einer vorherigen Wahrscheinlichkeitsaussage bestimmt wird, wobei das Fisher-Verhältnis für ein Gen j gegeben ist durch

$$F(j) = \frac{(\hat{\mu}_j(A) - \hat{\mu}_j(B))^2}{\hat{\sigma}_j^2(A) + \hat{\sigma}_j^2(B)}$$

wobei $\hat{\mu}_j(i)$ der Proben-Mittelwert des Expressionsspiegels des Gens j für die Proben des Grades i ist, und $\hat{\sigma}_j^2(i)$ die Proben-Abweichung des Expressionsspiegels des Gens j für die Proben des Grades i ist.

10. In vitro-Verfahren zur Definition des Differenzierungsgrads von Tumoren, wobei das Verfahren die Schritte umfasst:

(a) das Bestimmen der Zahl der Gene und/oder Proteine, um den Differenzierungsgrad von Tumoren zu definieren;
(b) das Auswählen einer Anzahl von Genen und/oder Proteinen, die in Schritt (a) bestimmt wurden, die die höchsten Fisher-Verhältnisse haben im Vergleich zwischen nicht-kanzeröser Leber und prä-kanzeröser Leber, prä-kanzeröser Leber und gut differenziertem Leberkarzinom (HCC), gut differenziertem HCC und gemäßigt differenziertem HCC, oder gemäßigt differenziertem HCC und schlecht differenziertem HCC;
(c) das Anwenden der in Schritt (b) ausgewählten Daten der Gene und/oder Proteine auf alle Proben; und
(d) das Definieren des Differenzierungsgrads von Tumoren,

wobei das Fisher-Verhältnis ohne die Verwendung einer vorherigen Wahrscheinlichkeitsaussage bestimmt wird, wobei das Fisher-Verhältnis für ein Gen j gegeben ist durch

$$F(j) = \frac{(\hat{\mu}_j(A) - \hat{\mu}_j(B))^2}{\hat{\sigma}_j^2(A) + \hat{\sigma}_j^2(B)}$$

wobei $\hat{\mu}_j(i)$ der Proben-Mittelwert des Expressionsspiegels des Gens j für die Proben des Grades i ist, und $\hat{\sigma}_j^2(i)$ die Proben-Abweichung des Expressionsspiegels des Gens j für die Proben des Grades i ist.

11. In vitro-Verfahren zum Definieren des Differenzierungsgrads von Tumoren, wobei das Verfahren die Schritte umfasst:

(a) das Bestimmen der Zahl der Gene und/oder Proteine, um den Differenzierungsgrad von Tumoren zu definieren;
(b) das Auswählen einiger Gene und/oder Proteine, die in Schritt (a) bestimmt wurden, die die höchsten Fisher-Verhältnisse haben im Vergleich zwischen nicht-kanzeröser Leber und prä-kanzeröser Leber, prä-kanzeröser Leber und gut differenziertem Leberkarzinom (HCCs), gut differenziertem HCC und gemäßigt differenziertem HCC, oder gemäßigt differenziertem HCC und schlecht differenziertem HCC;
(c) das Anwenden der in Schritt (b) ausgewählten Daten der Gene und/oder Proteine auf alle Proben;
(d) das Erstellen eines Mindestabstandsklassifikators mit den in Schritt (b) ausgewählten Daten der Gene und/oder Proteine;
(e) das Anwenden des in Schritt (d) erstellten Mindestabstandsklassifikators auf alle Proben;
(f) das Erzeugen einer Selbstorganisierenden Karte mit den in Schritt (b) ausgewählten Daten aller Gene und/oder Proteine;
(g) das Anwenden der in Schritt (f) erzeugten Selbstorganisierenden Karte auf alle Proben; und
(h) das Definieren des Differenzierungsgrads von Tumoren,

wobei das Fisher-Verhältnis ohne die Verwendung einer vorherigen Wahrscheinlichkeitsaussage bestimmt wird, wobei das Fisher-Verhältnis für ein Gen j gegeben ist durch

$$F(j) = \frac{(\hat{\mu}_j(A) - \hat{\mu}_j(B))^2}{\hat{\sigma}_j^2(A) + \hat{\sigma}_j^2(B)}$$

wobei $\hat{\mu}_j(i)$ der Proben-Mittelwert des Expressionsspiegels des Gens $j$ für die Proben des Grades $i$ ist, und $\hat{\sigma}_j^2(i)$ die Proben-Abweichung des Expressionsspiegels des Gens $j$ für die Proben des Grades $i$ ist.

**Revendications**

1. *Procédé in vitro* pour définir le degré de différenciation d'une tumeur avec des gènes et/ou protéines choisis par les analyses statistiques basées sur le taux ou le profil d'expression des gènes et/ou protéines de tissus tumoraux humains pouvant être obtenus à partir de patients cancéreux, dans lequel les gènes et/ou protéines sont choisis dans l'ordre décroissant du rapport de Fisher et dans lequel le rapport de Fisher est déterminé sans l'utilisation d'une probabilité antérieure, dans lequel le rapport de Fisher pour un gène j est donné par

$$F(j) = \frac{(\hat{\mu}_j(A) - \hat{\mu}_j(B))^2}{\hat{\sigma}_j^2(A) + \hat{\sigma}_j^2(B)}$$

dans lequel $\hat{\mu}_j(i)$ est la moyenne dans l'échantillon du taux d'expression du gène j pour les échantillons de degré $i$, et $\hat{\sigma}_j^2(i)$ est la variance dans l'échantillon du taux d'expression du gène j pour les échantillons de degré $i$.

2. *Procédé in vitro* selon la revendication 1, dans lequel les tissus humains sont des tissus de foie humain.

3. Procédé *in vitro* selon la revendication 2, dans lequel le degré de différenciation de la tumeur est choisi dans le groupe constitué par un foie non cancéreux, un foie précancéreux, un carcinome hépatocellulaire (HCC) bien différencié, un HCC modérément différencié et un HCC faiblement différencié.

4. Procédé *in vitro* selon la revendication 3, dans lequel les gènes et/ou protéines sont exprimés de manière différentielle entre un foie non cancéreux et un foie précancéreux, un foie précancéreux et un carcinome hépatocellulaire (HCC) bien différencié, un HCC bien différencié et un HCC modérément différencié, ou un HCC modérément différencié et un HCC faiblement différencié.

5. Procédé *in vitro* selon l'une quelconque des revendications 1 à 4, dans lequel le taux ou le profil d'expression des gènes et/ou protéines est étudié au moyen d'une micropuce à ADN, d'une réaction en chaîne par polymérase après transcription inverse ou d'une puce à protéines.

6. *Procédé in vitro* selon la revendication 5, dans lequel le nombre de gènes et/ou protéines est entre 40 et 100.

7. Procédé *in vitro* selon la revendication 5, dans lequel le nombre de gènes et/ou protéines est entre 35 et 45.

8. Procédé *in vitro* selon la revendication 7, dans lequel le nombre de gènes et/ou protéines est 40.

9. Procédé *in vitro* pour définir le degré de différenciation d'une tumeur, le procédé comprenant les étapes de :

   (a) sélection des gènes et/ou protéines qui présentent les rapports de Fisher les plus élevés par comparaison entre un foie non cancéreux et un foie précancéreux, un foie précancéreux et un carcinome hépatocellulaire (HCC) bien différencié, un HCC bien différencié et un HCC modérément différencié, ou un HCC modérément différencié et un HCC faiblement différencié ; et
   (b) définition du degré de différenciation d'une tumeur en utilisant les gènes et/ou protéines,

   dans lequel le rapport de Fisher est déterminé sans l'utilisation d'une probabilité antérieure, dans lequel le rapport du Fisher pour un gène j est donné par

$$F(j) = \frac{(\hat{\mu}_j(A) - \hat{\mu}_j(B))^2}{\hat{\sigma}_j^2(A) + \hat{\sigma}_j^2(B)}$$

dans lequel $\hat{\mu}_j(i)$ est la moyenne dans l'échantillon du taux d'expression du gène j pour les échantillons de degré $i$, et $\hat{\sigma}_j^2(i)$ est la variance dans l'échantillon du taux d'expression du gène j pour les échantillons de degré $i$.

**10.** Procédé *in vitro* pour définir le degré de différenciation d'une tumeur, le procédé comprenant les étapes de :

(a) détermination du nombre de gènes et/ou protéines pour définir le degré de différenciation d'une tumeur ;
(b) sélection d'un nombre décidé à l'étape (a) de gènes et/ou protéines qui présentent les rapports de Fisher les plus élevés par comparaison entre un foie non cancéreux et un foie précancéreux, un foie précancéreux et un carcinome hépatocellulaire (HCC) bien différencié, un HCC bien différencié et un HCC modérément différencié, ou un HCC modérément différencié et un HCC faiblement différencié ;
(c) application des données des gènes et/ou protéines choisis à l'étape (b) à tous les échantillons ; et
(d) définition du degré de différenciation d'une tumeur,

dans lequel le rapport de Fisher est déterminé sans l'utilisation d'une probabilité antérieure, dans lequel le rapport du Fisher pour un gène j est donné par

$$F(j) = \frac{(\hat{\mu}_j(A) - \hat{\mu}_j(B))^2}{\hat{\sigma}_j^2(A) + \hat{\sigma}_j^2(B)}$$

dans lequel $\hat{\mu}_j(i)$ est la moyenne dans l'échantillon du taux d'expression du gène j pour les échantillons de degré $i$, et $\hat{\sigma}_j^2(i)$ est la variance dans l'échantillon du taux d'expression du gène j pour les échantillons de degré $i$.

**11.** Procédé *in vitro* pour définir le degré de différenciation d'une tumeur, le procédé comprenant les étapes de :

(a) détermination du nombre de gènes et/ou protéines pour définir le degré de différenciation d'une tumeur ;
(b) sélection d'un nombre décidé à l'étape (a) de gènes et/ou protéines qui présentent les rapports de Fisher les plus élevés par comparaison entre un foie non cancéreux et un foie précancéreux, un foie précancéreux et un carcinome hépatocellulaire (HCC) bien différencié, un HCC bien différencié et un HCC modérément différencié, ou un HCC modérément différencié et un HCC faiblement différencié ;
(c) application des données des gènes et/ou protéines choisis à l'étape (b) à tous les échantillons ;
(d) conception d'un classificateur de distance minimale avec les données sur les gènes et/ou protéines choisis à l'étape (b) ;
(e) application du classificateur de distance minimale conçu à l'étape (d) à tous les échantillons ;
(f) génération d'une cartographie s'auto-organisant avec les données de tous les gènes et/ou protéines choisis à l'étape (b) ;
(g) application de la cartographie s'auto-organisant générée à l'étape (f) à tous les échantillons ; et
(h) définition du degré de différenciation de la tumeur,

dans lequel le rapport de Fisher est déterminé sans l'utilisation d'une probabilité antérieure, dans lequel le rapport du Fisher pour un gène j est donné par

$$F(j) = \frac{(\hat{\mu}_j(A) - \hat{\mu}_j(B))^2}{\hat{\sigma}_j^2(A) + \hat{\sigma}_j^2(B)}$$

dans lequel $\hat{\mu}_j(i)$ est la moyenne dans l'échantillon du taux d'expression du gène j pour les échantillons de degré $i$, et $\hat{\sigma}_j^2(i)$ est la variance dans l'échantillon du taux d'expression du gène j pour les échantillons de degré $i$.

Fig. 1a-d

Fig. 1e

* Fibronectin, Alt. Splice 1

Fig. 1f

Fig. 1g

Fig. 1h

Fig. 2

a

**L0 vs L1**

◇ L0
□ L1
△ G1
✕ G2
✳ G3

$T_1(x)$

b

**L1 vs G1**

◇ L0
□ L1
△ G1
✕ G2
✳ G3

$T_2(x)$

c

**G1 vs G2**

◇ L0
□ L1
△ G1
✕ G2
✳ G3

$T_3(x)$

d

**G2 vs G3**

◇ L0
□ L1
△ G1
✕ G2
✳ G3

$T_4(x)$

Training samples

Test samples

EP 1 613 767 B1

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03010337 A

**Non-patent literature cited in the description**

- **SCHAFER, D.F. ; SORRELL, M.F.** Hepatocellular carcinoma. *Lancet,* 1999, vol. 353, 1253-1257
- **COLOMBO, M.** Hepatitis C virus and hepatocellular carcinoma. *Semin. Liver Dis.,* 1999, vol. 19, 263-269
- **OKUDA, K.** Hepatocellular carcinoma. *J. Hepatol.,* 2000, vol. 32, 225-237
- **OKABE, H. ; SATOH, S. ; KATO, T. ; KITAHARA, O. ; YANAGAWA, R. ; YAMAOKA, Y. ; TSUNODA, T. ; FURUKAWA, Y. ; NAKAMURA, Y.** Genome-wide analysis of gene expression in human hepatocellular carcinomas using cDNA microarray: identification of genes involved in viral carcinogenesis and tumor progression. *Cancer Res,* 2001, vol. 61, 2129-2137
- **KOJIRO, M.** Pathological evolution of early hepatocellular carcinoma. *Oncology,* 2002, vol. 62, 43-47
- Quantitative monitoring of gene expression patterns with a complementary DNA microarray. *Science,* 1975, vol. 270, 467-470
- **DERISI, J. ; PENLAND, L. ; BROWN, P.O. ; BITTNER, M.L. ; MELTZER, P.S. ; RAY, M. ; CHEN, Y. ; SU, Y . A ; TRENT, J.M.** Use of a cDNA microarray to analyse gene expression patterns in human cancer. *Nat. Genet.,* 1996, vol. 14, 457-460
- **LAU, W.Y. ; LAI, P.B. ; LEUNG, M.F. ; LEUNG, B.C. ; WONG, N. ; CHEN, G. ; LEUNG, T.W. ; LIEW, C.T.** Differential gene expression of hepatocellular carcinoma using cDNA microarray analysis. *Oncol. Res.,* 2000, vol. 12, 59-69
- **TACKELS-HORNE, D. ; GOODMAN, M.D. ; WILLIAMS, A.J. ; WILSON, D.J. ; ESKANDARI, T. ; VOGT, L.M. ; BOLAND, J.F. ; SCHERF, U. ; VOCKLEY, J.G.** Identification of differentially expressed genes in hepatocellular carcinoma and metastatic liver tumors by oligonucleotide expression profiling. *Cancer,* 2001, vol. 92, 395-405
- **XU, L. ; HUI, L. ; WANG, S. ; GONG, J. ; JIN, Y. ; WANG, Y. ; JI, Y. ; WU, X. ; HAN, Z. ; HU, G.** Expression profiling suggested a regulatory role of liver-enriched transcription factors in human hepatocellular carcinoma. *Cancer Res.,* 2001, vol. 61, 3176-3681

- **XU, X.R. ; HUANG, J. ; XU, Z.G. ; QIAN, B.Z. ; ZHU, Z. D. ; YAN, Q. ; CAI, T. ; ZHANG, X. ; XIAO, H.S. ; QU, J.** Insight into hepatocellular carcinogenesis at transcriptome level by comparing gene expression profiles of hepatocellular carcinoma with those of corresponding non-cancerous liver. *Proc. Natl. Acad. Sci. U.S.A.,* 2001, vol. 98, 15089-15094
- **OKABE, H. ; SATOH, S. ; KATO, T. ; KITAHARA, O. ; YANAGAWA, R. ; YAMAOKA, Y. ; TSUNODA, T. ; FURUKAWA, Y. ; NAKAMURA, Y.** Genome-wide analysis of gene expression in human hepatocellular carcinomas using cDNA microarray: identification of genes involved in viral carcinogenesis and tumor progression. *Cancer Res.,* 2001, vol. 61, 2129-2137
- **SHIROTA, Y. ; KANEKO, S. ; HONDA, M. ; KAWAI, H.F. ; KOBAYASHI, K.** Identification of differentially expressed genes in hepatocellular carcinoma with cDNA microarrays. *Hepatology,* 2001, vol. 33, 832-840
- **DELPUECH, O. ; TRABUT, J.B. ; CARNOT, F. ; FEUILLARD, J. ; BRECHOT, C. ; KREMSDORF, D.** Identification, using cDNA macroarray analysis, of distinct gene expression profiles associated with pathological and virological features of hepatocellular carcinoma. *Oncogene,* 2002, vol. 21, 2926-2937
- **IIZUKA, N. ; OKA, M. ; YAMADA-OKABE, H. ; MORI, N. ; TAMESA, T. ; OKADA, T. ; TAKEMOTO, T. ; TANGOKU, A. ; HAMADA, K. ; NAKAYAMA, H.** Comparison of gene expression profiles between hepatitis B virus- and hepatitis C virus-infected hepatocellular carcinoma by oligonucleotide microarray data based on a supervised learning method. *Cancer Res.,* 2002, vol. 62, 3939-3944
- **MIDORIKAWA, Y. ; TSUTSUMI, S. ; TANIGUCHI, H. ; ISHII, M. ; KOBUNE, Y. ; KODAMA, T. ; MAKUUCHI, M. ; ABURATANI, H.** Identification of genes associated with dedifferentiation of hepatocellular carcinoma with expression profiling analysis. *Jpn. J. Cancer Res.,* 2002, vol. 93, 636-643
- **IIZUKA et al.** *Cancer Research,* 2002, vol. 62, 3939-3944

- **TAMAYO et al.** *Proc. Natl. Acad. Sci. USA,* 1999, vol. 96, 2907-2912
- **TÖRÖNEN et al.** *FEBS letters,* 1999, vol. 451, 142-146
- **EDMONDSON, H.A. ; STEINER, P.E.** Primary carcinoma of the liver: a study of 100 cases among 48,900 necropsies. *Cancer,* 1954, vol. 7, 462-504
- **SCHENA, M. et al.** Quantitative monitoring of gene expression patterns with a complementary DNA microarray. *Science,* 1995, vol. 270, 467-470
- **LIPSHUTZ, R.J. et al.** High density synthetic oligonucleotide arrays. *Nat. Genet.,* 1999, vol. 21, 20-24
- **WEIS, J.H. et al.** Detection of rare mRNAs via quantitative RT-PCR. *Trends Genet.,* 1992, vol. 8, 263-264
- **BUSTIN, S.A.** Absolute quantification of mRNA using real-time reverse transcription polymerase chain reaction assays. *J. Mol. Endocrinol.,* 2000, vol. 25, 169-193
- **PARKER, R.M. ; BARNES, N.M.** mRNA: detection in situ and northern hybridization. *Methods Mol. Biol.,* 1999, vol. 106, 247-283
- **HOD, Y.A.** Simplified ribonuclease protection assay. *BioTechniques,* 1992, vol. 13, 852-854
- **SACCOMANNO, C.F. et al.** A faster ribonuclease protection assay. *BioTechniques,* 1992, vol. 13, 846-850
- **TOWBIN, H. ET AL.** Electrophoretic transfer of proteins from polyacrylamide gels to nitrocellulose sheets. *Proc. Natl. Acad. Sci. U. S.A.,* 1979, vol. 76, 4350-4354
- **BURNETTE, W.N.** Western blotting: Electrophoretic transfer of proteins form sodium dodecyl sulfate-polyacrylamide gels to unmodified nitrocellulose and radioiodinated protein A. *Anal. Biochem.,* 1981, vol. 112, 195-203
- **ENGVALL, E. ; PERLMAN, P.** Enzyme-linked immunosorbent assay (ELISA) : Quantitative assay of immunoglobulin G. *Immunochemistry,* 1971, vol. 8, 871-879
- **MERCHANT, M. ; WEINBERGER, S.R.** Review: Recent advancements in surface-enhanced laser desorption/ionization-time of flight-mass spectrometry. *Electrophoresis,* 2000, vol. 21, 1164-1177
- **PAWELETZ, C.P. et al.** Rapid protein display profiling of cancer progression directly from human tissue using a protein biochip. *Drug Dev. Res.,* 2000, vol. 49, 34-42
- **TAMAYO, P. et al.** Interpreting patterns of gene expression with self-organizing maps: methods and application to hematopoietic differentiation. *Proc. Natl. Acad. Sci. U.S.A.,* 1999, vol. 96, 2907-2912
- **SULTAN, M. et al.** Binary tree-structured vector quantization approach to clustering and visualizing microarray data. *Bioinformatics,* 2002, vol. 1, S111-S119
- **LIPSHUTZ, R.L. et al.** High density synthetic oligonucleotide arrays. *Nat. Genet.,* 1999, vol. 21, 20-24
- **LUO, J. ; DUGGAN, D.J. ; CHEN, Y. ; SAUVAGEOT, J. ; EWING, C.M. ; BITTNER, M.L. ; TRENT, J.M. ; ISAACS, W.B.** Human prostate cancer and benign prostatic hyperplasia: molecular dissection by gene expression profiling. *Cancer Res,* 2001, vol. 61, 4683-4688
- **LETO, G. ; TUMMINELLO, F.M. ; PIZZOLANTI, G. ; MONTALTO, G. ; SORESI, M. ; RUGGERI, I. ; GEBBIA, N.** Cathepsin D serum mass concentrations in patients with hepatocellular carcinoma and/or liver cirrhosis. *Eur. J. Clin. Chem. Clin. Biochem.,* 1996, vol. 34, 555-560
- **YOSHII, J. ; YOSHIJ I, H. ; KURIYAMA, S. ; IKENAKA, Y. ; NOGUCHI, R. ; OKUDA, H. ; TSUJINOUE, H. ; NAKATANI, T. ; KISHIDA, H. ; NAKAE, D.** The copper-chelating agent, trientine, suppresses tumor development and angiogenesis in the murine hepatocellular carcinoma cells. *Int. J. Cancer.,* 2001, vol. 94, 768-773
- **MOUTA CARREIRA, C. ; NASSER, S.M. ; DI TOMASO, E. ; PADERA, T.P. ; BOUCHER, Y. ; TOMAREV, S.I. ; JAIN, R.K.** LYVE-1 is not restricted to the lymph vessels: expression in normal liver blood sinusoids and down-regulation in human liver cancer and cirrhosis. *Cancer Res,* 2001, vol. 61, 8079-8084
- **IIZUKA, N. ; OKA, M. ; YAMADA-OKABE, H. ; MORI, N. ; TAMESA, T. ; OKADA, T. ; TAKEMOTO, T. ; TANGOKU, A. ; HAMADA, K. ; NAKAYAMA, H.** Comparison of gene expression profiles between hepatitis B virus- and hepatitis C virus-infected hepatocellular carcinoma by oligonucleotide microarray data based on a supervised learning method. *Cancer Res,* 2002, vol. 62, 3939-3944
- **SHOU, J. ; SORIANO, R. ; HAYWARD, S.W. ; CUNHA, G.R. ; WILLIAMS, P.M. ; GAO, W.Q.** Expression profiling of a human cell line model of prostatic cancer reveals a direct involvement of interferon signaling in prostate tumor progression. *Proc. Natl. Acad. Sci. U.S.A.,* 2002, vol. 99, 2830-2835
- **BROMBERG, J.F.** Activation of STAT proteins and growth control. *Bioessays,* 2001, vol. 23, 161-169
- **RADAEVA, S. ; JARUGA, B. ; HONG, F. ; KIM, W.H. ; FAN, S. ; CAI, H. ; STROM, S. ; LIU, Y. ; EL-ASSAL, O. ; GAO, B.** Interferon-alpha activates multiple STAT signals and down-regulates c-Met in primary human hepatocytes. *Gastroenterology,* 2002, vol. 122, 1020-1034
- **HUNG, W.C. ; CHUANG, L.Y.** Sodium butyrate enhances STAT 1 expression in PLC/PRF/5 hepatoma cells and augments their responsiveness to interferon-alpha. *Br. J. Cancer,* 1999, vol. 80, 705-710

- **SPAGNOLI, A. ; TORELLO, M. ; NAGALLA, S.R. ; HORTON, W.A. ; PATTEE, P. ; HWA, V. ; CHIARELLI, F. ; ROBERTS, C.T. JR.** Rosenfeld, R.G. Identification of STAT-1 as a molecular target of IGFBP-3 in the process of chondrogenesis. *J. Biol. Chem.,* 2002, vol. 277, 18860-18867
- **NAGATA, K. ; HIRONO, S. ; IDO, A. ; KATAOKA, H. ; MORIUCHI, A. ; SHIMOMURA, T. ; HORI, T. ; HAYASHI, K. ; KOONO, M. ; KITAMURA, N.** Expression of hepatocyte growth factor activator and hepatocyte growth factor activator inhibitor type 1 in human hepatocellular carcinoma. *Biochem. Biophys. Res. Commun.,* 2001, vol. 289, 205-211

- **OHANNESIAN, D.W. ; LOTAN, D. ; THOMAS, P. ; JESSUP, J.M. ; FUKUDA, M. ; GABIUS, H.J. ; LOTAN, R.** Carcinoembryonic antigen and other glycoconjugates act as ligands for galectin-3 in human colon carcinoma cells. *Cancer Res,* 1995, vol. 55, 2191-2199